# EUROPEAN PATENT SPECIFICATION

(11) **EP 2 464 728 B1**
(45) Date of publication and mention of the grant of the patent: **12.04.2017**
(21) Application number: 10749501.2
(22) Date of filing: 12.08.2010
(51) Int. Cl.: C12N 15/10, C40B 40/06, C12N 15/62

(54) **A RATIONAL LIBRARY**
RATIONALE BIBIOTHEK
BIBLIOTHÈQUE RATIONNELLE

(30) Priority: 12.08.2009 US 233294 P
(43) Date of publication of application: 20.06.2012
(73) Proprietor: UNI TARGETING RESEARCH AS, 5006 Bergen (NO)
(72) Inventor: STERN, Beate, N-5099 Bergen (NO); PRYME, Ian, Fraser, N-5098 Bergen (NO); RAVNEBERG, Hanne, N-5009 Bergen (NO)
(74) Representative: Rowe, Daniel
(86) International application number: PCT/IB2010/053648
(87) International publication number: WO 2011/018766

(56) References cited:
- WO-A1-2009/020899
- WO-A2-2005/001099
- KLEIN B K ET AL: "EFFECTS OF SIGNAL PEPTIDE CHANGES ON THE SECRETION OF BOVINE SOMATOTROPIN (BST) FROM ESCHERICHIA COLI" PROTEIN ENGINEERING, OXFORD UNIVERSITY PRESS, SURREY, GB, vol. 5, no. 6, 1 September 1992 (1992-09-01), pages 511-517, XP009006075 ISSN: 0269-2139
- STERN, B. ET AL: "Improving mammalian cell factories : The selection of signal peptide has a major impact on recombinant protein synthesis and secretion in mammalian cells." TRENDS CELL MOL. BIOL., vol. 2, 1 January 2007 (2007-01-01), pages 1-17, XP002603748
- KNAPPSKOG ET AL: "The level of synthesis and secretion of Gaussia princeps luciferase in transfected CHO cells is heavily dependent on the choice of signal peptide" JOURNAL OF BIOTECHNOLOGY, ELSEVIER SCIENCE PUBLISHERS, AMSTERDAM, NL LNKD- DOI:10.1016/J.JBIOTEC.2006.11.026, vol. 128, no. 4, 6 March 2007 (2007-03-06) , pages 705-715, XP005916232 ISSN: 0168-1656
- TRÖSSE CHRISTIANE ET AL: "Vectors encoding seven oikosin signal peptides transfected into CHO cells differ greatly in mediating Gaussia luciferase and human endostatin production although mRNA levels are largely unaffected." 2007, GENE REGULATION AND SYSTEMS BIOLOGY : 2007 LNKD- PUBMED:19936096, VOL. 1, PAGE(S) 303 - 312 , XP002603749 ISSN: 1177-6250 page 305, right-hand column, last paragraph - page 306, left-hand column, paragraph 1; figure 1; table 1 page 309, right-hand column, last paragraph - page 311, left-hand column, paragraph 3
- ZHANG L ET AL: "Alteration in the IL-2 signal peptide affects secretion of protein in vitro and in vivo" JOURNAL OF GENE MEDICINE, WILEY, US LNKD- DOI:10.1002/JGM.677, vol. 7, no. 3, 1 March 2005 (2005-03-01), pages 354-365, XP003015830 ISSN: 1099-498X

### SEQUENCE LISTING

<110> UTR
   UniTargeting Research AS
<120> Rational library
<130> P8838PC00
<150> US61/233294
   <151> 2009-08-12
<160> 14
<170> PatentIn version 3.5
<210> 1
   <211> 51
   <212> DNA
   <213> Signal sequence derived from Metridia longa luciferase with mutations
<400> 1
   atggatataa aggttgtctt tgcccttgtt ttctgcgcat tggttcaggc a 51
<210> 2
   <211> 48
   <212> DNA
   <213> Signal sequence derived from Oikopleura dioica oikosin 1 with mutation
<400> 2
   atgctgctgt tggttgcgct tctccttggg cttgcccatg ggtactcc 48
<210> 3
   <211> 48
   <212> DNA
   <213> Signal sequence derived from Oikopleura dioica oikosin 1 with mutation
<400> 3
   atgctgctgt tggttgcgct tctccttggg cttgccgctg ggtactcc 48
<210> 4
   <211> 48
   <212> DNA
   <213> Signal sequence derived from Oikopleura dioica oikosin 1 with mutation
<400> 4
   atgctgctgc tgtccgccct gctgctggga ctggctgccg gctacagc 48
<210> 5
   <211> 54
   <212> DNA
   <213> Signal sequence derived from Homo sapiens chymotrypsinogen B2
<400> 5
   atggctttcc tctggctcct ctcctgctgg gccctcctgg gtaccacctt cggc 54
<210> 6
   <211> 51
   <212> DNA
   <213> Codon optimised signal sequence derived from Gaussia princeps
   luciferase
<400> 6
   atgggagtca aagttctgtt tgccctgatc tgcatcgctg tggccgaggc c 51
<210> 7
   <211> 54
   <212> DNA
   <213> Signal sequence derived from Homo sapiens chymotrypsinogen B2 with
   mutations
<400> 7
   atggctttcc tctggctcct cttctgctgg gccctcctgg gtaccacctt cggc 54
<210> 8
   <211> 48
   <212> DNA
   <213> Signal sequence derived from Oikopleura dioica oikosin 1 with mutation
<400> 8
   atgctgctgt tgtcagcgct tctccttggg cttgcctttg ggtactcc 48
<210> 9
   <211> 48
   <212> DNA
   <213> Signal sequence derived from Oikopleura dioica oikosin 1
<400> 9
   atgctgctgt tgtcagcgct tctccttggg cttgcccatg ggtactcc 48
<210> 10
   <211> 51
   <212> DNA
   <213> Signal sequence derived from Metridia longa luciferase
<400> 10
   atggatataa aggttgtctt tactcttgtt ttctcagcat tggttcaggc a 51
<210> 11
   <211> 51
   <212> DNA
   <213> Codon optimised signal sequence derived from Gaussia princeps
   luciferase
<400> 11
   atgggcgtga aggtgctgtt cgccctgatc tgcatcgccg tggccgaggc c 51
<210> 12
   <211> 57
   <212> DNA
   <213> Signal sequence derived from Mus musculus immunoglobulin lambda-1
   light chain precursor
<400> 12
   atggcctgga tttcacttat actctctctc ctggctctca gctcaggggc catttcc 57
<210> 13
   <211> 74
   <212> DNA
   <213> Mutagenic primer used for Gluc SS randomisation at the positions
   indicated by N's
<220>
   <221> misc_feature
   <222> (27)..(35)
   <223> n is a, c, g, or t
<220>
   <221> misc_feature
   <222> (54)..(56)
   <223> n is a, c, g, or t
<400> 13
   caaagtatct tctggcaggg aaaatgnnnn nnnnngttct gtttgccctg atcnnnatcg 60
   ctgtggccga ggcc 74
<210> 14
   <211> 74
   <212> DNA
   <213> Mutagenic primer used for LC SS randomisation at the positions
   indicated by N's
<220>
   <221> misc_feature
   <222> (33)..(38)
   <223> n is a, c, g, or t
<220>
   <221> misc_feature
   <222> (48)..(50)
   <223> n is a, c, g, or t
<400> 14
   caaagtatct tctggcaggg aaaatggcct ggnnnnnnct tatactcnnn ctcctggctc    60
   tcagctcagg ggcc                                                      74

## Description

### FIELD OF INVENTION

The invention relates to a method for the increased production of a recombinant protein of interest. Said method involves the generation and use of rational libraries comprising genetic elements which are involved in transcriptional and/or translational regulation of a gene and devised to increase the production yield of the encoded protein.

### BACKGROUND OF INVENTION

The number of recombinant proteins used for therapeutic and research purposes has increased substantially during the last decades, and the demand is expected to further increase dramatically in the future. The most commonly commercially produced proteins are growth factors, antibodies, enzymes, hormones and blood factors. Monoclonal antibody (MAb) therapeutics are set to play a significant role in the treatment of a wide number of diseases with cancer, arthritis, immune and inflammatory diseases as the main focus. Today MAb production shows the strongest growth area of therapeutic proteins.

Recombinant proteins can be produced in various cell expression systems, each having its advantages and drawbacks. Bacterial systems have the advantages of easy handling, rapid growth and high-yield protein production at relatively low-costs, but lack the post-translational modification (PTM) machinery found in eukaryotes. For production of more complex molecules, such as glycosylated proteins more advanced cell systems are required, such as eukaryotic systems.

Today mammalian cell systems provide the best opportunity to produce safe and fully biologically active glycosylated proteins, despite their bottleneck of high costs, complicated process technology, and the potential risk of carrying animal viruses.

The classical manufacturing process to generate a stable cell line producing the recombinant protein requires that an expression vector containing the gene of interest together with a selection marker gene must be generated; this vector is then introduced into the cells which are selected for the presence of the marker protein. Single cells surviving selection are expanded to clonal cell lines which are screened for high-yield recombinant protein production. A cell line suitable for industrial production requires three key criteria: a high growth rate, high specific productivity regarding the recombinant protein, and the ability of maintaining high titers over an extended period of time.

In order to accomplish yields up to the biological limit, there is still room for drastic improvements of mammalian cell systems, e.g. by genetic engineering of expression vectors, such that the recombinant protein in question is efficiently synthesised and secreted in larger quantities than currently possible using available technology.

Virtually all recombinant proteins that are in current use are naturally secreted. Such proteins are initially synthesised with a small N-terminal peptide - the signal peptide (SP). The SP is of importance in the process of co-translational translocation of the growing polypeptide chain whereby the molecule is effectively transferred into the lumen of the endoplasmic reticulum (ER). Having achieved this, the SP is cleaved off the growing polypeptide chain by the enzyme signal peptidase. The synthesised protein is then transported through the ER to the Golgi apparatus where appropriate PTM occurs. An important PTM is glycosylation, a necessary prerequisite for the expression of biological activity of many secreted proteins e.g. MAbs.

A very important genetic element that has to be present in a vector to be used for the expression of a gene coding for a secreted protein is thus the signal sequence (SS) - the stretch of nucleotides that codes for the SP. There has hitherto been little attention addressed to this sequence with respect to enhanced protein production. It has recently become evident to us that the actual choice of SP has a large impact on the level of synthesis/secretion of the protein of choice (Knappskog *et al.* 2007; Stern *et al.* 2007; Tröße et al. 2007). Importantly, there appears to be no "universal SP" that can be used for all recombinant proteins (Stern *et al.* 2007).

The "individual" requirement of each protein to be produced can be extended to include other genetic regulatory elements in a vector involved in transcription and translation, such as 5' and 3' untranslated regions (5'UTR and 3'UTR), intron and promoter. To address this issue a novel, intelligent approach is needed. One efficient way is to create rational genetic libraries containing one or more of the genetic elements carrying mutations in one or more pre-defined positions. Generating genetic libraries where all positions of an element are randomised is not a viable approach, since the number of variants will be too huge to allow for identification of the best ones. For example, a total random 20 amino acids (AAs) long SP would give approximately 10²⁶ possible variants at the AA level and 10³⁶ at the DNA level.

### SUMMARY OF THE INVENTION

There is described herein a method to design a rational library containing genetic elements, wherein said library may be used to screen for clones that result in an increased expression of a gene of interest, wherein the increase is due to that one or more nucleotides at specific positions have been randomised in one of the genetic elements involved in transcriptional and/or translational regulation of a gene. The genetic library is developed using a strategy which combines the selection of specific positions within any of these elements with randomisation of nucleotides at these predetermined positions.

By such a strategy and subsequent transfection of a host cell line with this library a cell pool comprising a large, though limited number of clones will be generated. This will provide the opportunity of finding clones that produce the encoded recombinant protein of interest at much higher levels than would be otherwise obtained.

By this method in which specific nucleotide positions are pre-defined as being of interest for randomisation, a library approach becomes feasible. By identifying and using specific "high-impact positions", the number of different variants will be greatly reduced as compared to employing a library generated by complete genetic element randomisation, though still ensuring that the best variants are included. Therefore the probability of finding the best performing variant in a screening procedure is much higher than when having to find it in a much bigger background where complete screening would be impractical.

More specifically there is described herein a method to generate rational libraries comprising genetic elements involved in transcriptional and/or translational regulation of a gene and devised to increase the production yield of the encoded protein, comprising the steps of: providing a genetic element to be optimised for expression capacity and defining at most 18 nucleotide residues, either non-coding or coding for at most 6 amino acid residues, at specific positions in said genetic element to be randomised, amplifying said genetic element, said genetic element being part of a double stranded DNA plasmid being a preliminary vector or a final vector and subjecting said genetic element to randomisation and generating a pool of genetic element variants, amplifying said pool of genetic element variants being part either of a preliminary vector, thus generating a pre-made library or being part of a final vector, thus generating a final library, or introducing said pool of genetic element variants being part of a preliminary vector into a recipient vector in a seamless manner, thus generating a final library, transforming said final library into eukaryotic cells and obtaining a eukaryotic cell pool containing a rational library comprising up to 4¹⁸ ≈ 6.9x10¹⁰ different vector variants.

By such a rational library approach it will for the first time enable one to identify among many clones containing different genetic element variants, wherein one genetic element has been subjected to randomisation in at most 18 nucleotide positions, the best-performing clone that produces the encoded recombinant protein of interest to a substantially higher level than that mediated by the original non-modified nucleotide sequence, with a very high likelihood. Thus the approach is both efficient and guarantees a high success rate.

Also described herein is a method to identify a clonal cell line within a cell pool, harbouring a vector variant where said clonal cell line produces a protein of interest at the highest amount, comprising the steps of generating the genetic element variants in a vector containing the gene encoding the protein of interest or incorporating said genetic element variants from a pre-made library into a vector containing the gene encoding the protein of interest or incorporating the gene encoding the protein of interest into a pre-made library according to what is described in the application, screening for the cell clone that produces the protein of interest to the highest level and obtaining a clonal cell line from the cells transfected with the rational library, giving rise to the highest level of production of the encoded protein.

Such a method to identify a clonal cell line that produces the encoded recombinant protein of interest to higher levels compared to a cell line not having been transfected with a vector exposed to specific nucleotide randomisation within its genetic elements, will result in identifying such a clone with a very high level of probability and thereby the production of biologics, biosimilars, industrial proteins, proteins for research or any other protein of interest can be significantly increased.

Also described herein is a rational library based on a vector containing different genetic elements which have been seamlessly cloned, said rational library containing up to 7x10¹⁰ different vector variants wherein each variant contains at most 18 randomised nucleotides, either non-coding or coding for at most 6 amino acid residues, at specific positions of one of the genetic elements and wherein each vector variant mediates a different expression level of the encoded protein of interest as compared to the non-modified vector.

More specifically the invention relates to the use of the above described methods as well as the rational library for the increased production of recombinant proteins in a eukaryotic cell.

Thus in a first aspect the invention specifically provides a method for the increased production of a recombinant protein of interest in a eukaryotic cell, said method comprising
(i) providing a eukaryotic cell pool containing a final rational library of up to 4¹⁸ different vector variants containing randomised variants of a signal sequence and a gene encoding the recombinant protein of interest, said eukaryotic cell pool being obtained by a method comprising
   (a) providing said signal sequence and defining at most 18 nucleotide residues coding for at most 6 amino acid residues at specific positions in said signal sequence to be randomised; and
   (b) amplifying said signal sequence as part of a double stranded DNA plasmid, wherein said plasmid may be a preliminary vector, or a final vector containing a gene encoding the recombinant protein of interest, and subjecting said signal sequence to randomisation at said specified positions thereby generating a pool of randomised variants of said signal sequence as part of a vector; and
   (ci) amplifying said pool of randomised variants of said signal sequence being part of a preliminary vector, thereby generating a pre-made rational library, and introducing said pool of randomised variants of said signal sequence being part of the pre-made rational library into a recipient vector containing a gene encoding the recombinant protein in a seamless manner, thereby generating the final rational library, or
   (cii) amplifying said pool of randomised variants of said signal sequence being part of a preliminary vector, thereby generating a pre-made rational library, and introducing a gene encoding the recombinant protein into the pre-made rational library in a seamless manner, thereby generating the final rational library, or
   (ciii) amplifying said pool of randomised variants of said signal sequence being part of a final vector, thereby generating the final rational library; and
   (d) transforming said final rational library into eukaryotic cells thereby obtaining said eukaryotic cell pool; and
(ii) culturing said cell pool under conditions suitable for the production of said recombinant protein of interest; and
(iii) identifying within said cell pool a cell clone that produces the recombinant protein of interest at a higher level than that mediated by the unmodified signal sequence.

In a preferred embodiment of this aspect the invention provides a method for the increased production of a recombinant protein of interest in a eukaryotic cell, said method comprising
(i) providing a signal sequence and defining at most 18 nucleotide residues coding for at most 6 amino acid residues at specific positions in said signal sequence to be randomised; and
(ii) amplifying said signal sequence as part of a double stranded DNA plasmid, wherein said plasmid may be a preliminary vector, or a final vector containing a gene encoding the recombinant protein of interest, and subjecting said signal sequence to randomisation at said specified positions thereby generating a pool of randomised variants of said signal sequence as part of a vector; and
   (iiia) amplifying said pool of randomised variants of said signal sequence being part of a preliminary vector, thereby generating a pre-made rational library, and introducing said pool of randomised variants of said signal sequence being part of the pre-made rational library into a recipient vector containing a gene encoding the recombinant protein in a seamless manner, thereby generating a final rational library of up to 4¹⁸ different vector variants containing randomised variants of said signal sequence and the gene encoding the recombinant protein of interest, or
   (iiib) amplifying said pool of randomised variants of said signal sequence being part of a preliminary vector, thereby generating a pre-made rational library, and introducing a gene encoding the recombinant protein into the pre-made rational library in a seamless manner, thereby generating a final rational library of up to 4¹⁸ different vector variants containing randomised variants of said signal sequence and the gene encoding the recombinant protein of interest, or
   (iiic) amplifying said pool of randomised variants of said signal sequence being part of a final vector, thereby generating a final rational library of up to 4¹⁸ different vector variants containing randomised variants of said signal sequence and the gene encoding the recombinant protein of interest; and
(iv) transforming said final rational library into eukaryotic cells thereby obtaining a eukaryotic cell pool containing the final rational library; and
(v) culturing said cell pool under conditions suitable for the production of said recombinant protein of interest; and
(vi) identifying within said cell pool a cell clone that produces the recombinant protein of interest at a higher level than that mediated by the unmodified signal sequence.

In a further preferred embodiment of this aspect the invention provides a method for the increased production of a recombinant protein of interest in a eukaryotic cell, wherein said method comprises
(i) providing a pre-made rational library of up to 4¹⁸ different vector variants containing randomised variants of a signal sequence as part of a preliminary vector, said pre-made rational library being obtained by a method comprising:
   (a) providing said signal sequence and defining at most 18 nucleotide residues coding for at most 6 amino acid residues at specific positions in said signal sequence to be randomised; and
   (b) amplifying said signal sequence as part of a double stranded DNA plasmid being a preliminary vector and subjecting said signal sequence to randomisation at said specified positions thereby generating a pool of randomised variants of said signal sequence as part of a preliminary vector; and
   (c) amplifying said pool of randomised variants of said signal sequence as part of a preliminary vector, thereby generating said pre-made rational library; and
(iia) introducing said randomised variants of said signal sequence of said pre-made rational library into a recipient vector containing a gene encoding the recombinant protein in a seamless manner, thereby generating a final rational library of up to 4¹⁸ different vector variants containing randomised variants of said signal sequence and the gene encoding the recombinant protein of interest, or
(iib) introducing a gene encoding the recombinant protein into the pre-made rational library in a seamless manner, thereby generating a final rational library of up to 4¹⁸ different vector variants containing randomised variants of said signal sequence and the gene encoding the recombinant protein of interest; and
(iii) transforming said final rational library into eukaryotic cells thereby obtaining a eukaryotic cell pool containing the final rational library; and
(iv) culturing said cell pool under conditions suitable for the production of said recombinant protein of interest; and
(v) identifying within said cell pool a cell clone that produces the recombinant protein of interest at a higher level than that mediated by the unmodified signal sequence. In a still further preferred embodiment the invention provides a method for the increased production of a recombinant protein of interest in a eukaryotic cell, wherein said method comprises
   (i) providing a signal sequence and defining at most 18 nucleotide residues coding for at most 6 amino acid residues at specific positions in said signal sequence to be randomised; and
   (ii) amplifying said signal sequence as part of a double stranded DNA plasmid being a preliminary vector and subjecting said signal sequence to randomisation at said specified positions thereby generating a pool of randomised variants of said signal sequence as part of a preliminary vector; and
   (iii) amplifying said pool of randomised variants of said signal sequence as part of a preliminary vector, thereby generating a pre-made rational library of up to 4¹⁸ different vector variants containing randomised variants of said signal sequence as part of a preliminary vector; and
      (iva) introducing said randomised variants of said signal sequence of said pre-made rational library into a recipient vector containing a gene encoding the recombinant protein in a seamless manner, thereby generating a final rational library of up to 4¹⁸ different vector variants containing randomised variants of said signal sequence and the gene encoding the recombinant protein of interest, or
      (ivb) introducing a gene encoding the recombinant protein into the pre-made rational library in a seamless manner, thereby generating a final rational library of up to 4¹⁸ different vector variants containing randomised variants of said signal sequence and the gene encoding the recombinant protein of interest; and
   (v) transforming said final rational library into eukaryotic cells thereby obtaining a eukaryotic cell pool containing the final rational library; and
   (vi) culturing said cell pool under conditions suitable for the production of said recombinant protein of interest; and
   (vii) identifying within said cell pool a cell clone that produces the recombinant protein of interest at a higher level than that mediated by the unmodified signal sequence.

### BRIEF DESCRIPTION OF THE DRAWINGS

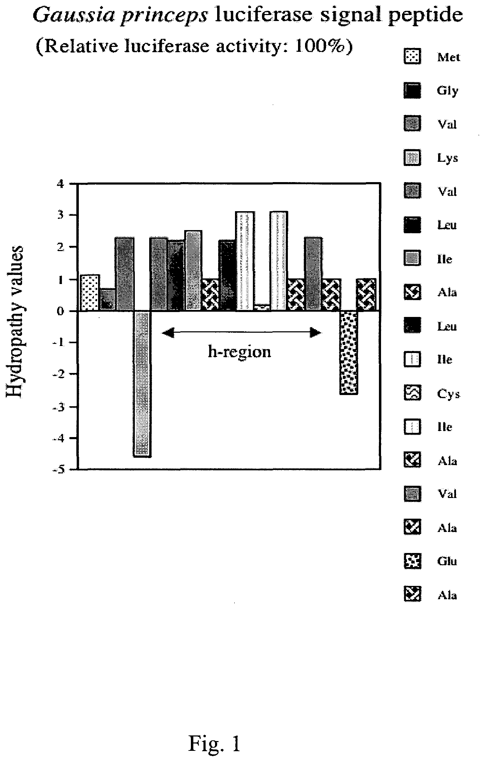

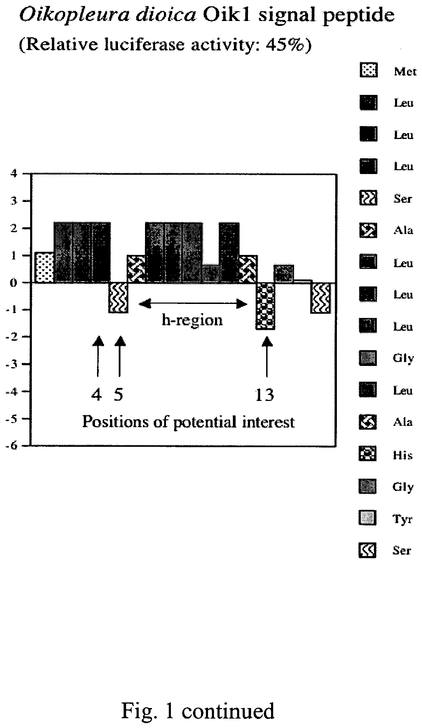

Figure 1 shows the hydropathy plots of the SPs of *Gaussia princeps* luciferase (left panel) and Oik1 from *Oikopleura dioica* (right panel). Hydropathy scores are taken from Eisenberg *et al.* (1982). Vertical arrows in the right panel indicate AA positions judged to be of high impact regarding the performance of Oik1 SP.

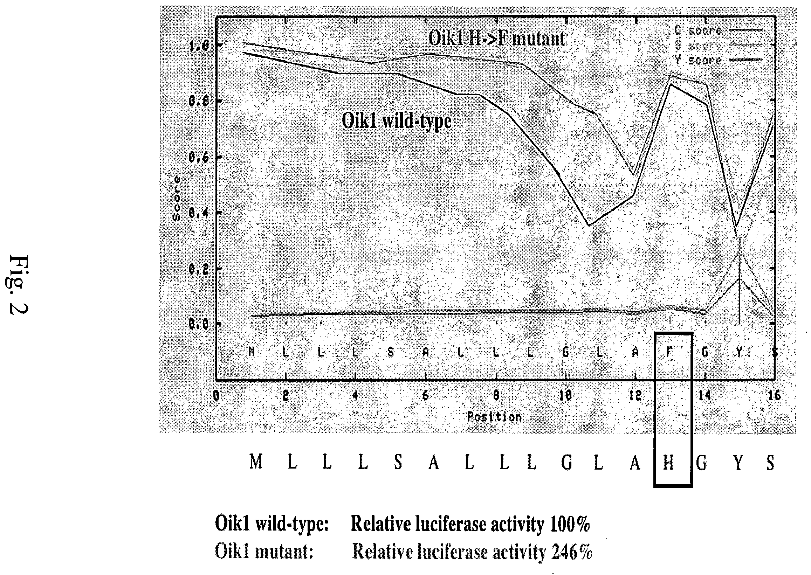

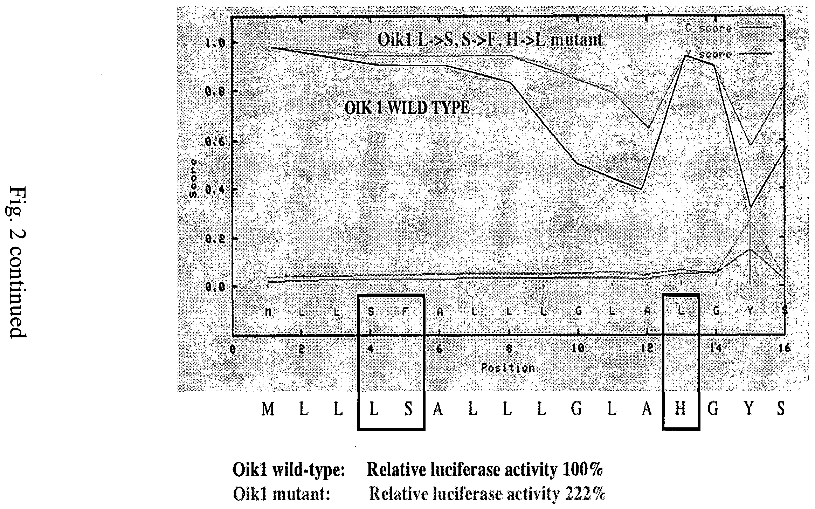

Figure 2 shows the S-score plot of Oik1 SP wild-type superimposed on the S-score plot of the H15F mutant of Oik1 SP (upper panel) and of the L4S, S5F, H13L mutant of Oik1 SP (lower panel). The plots were generated by using the SignalP 3.0 Server (http://www.cbs.dtu.dk/services/SignalP/).

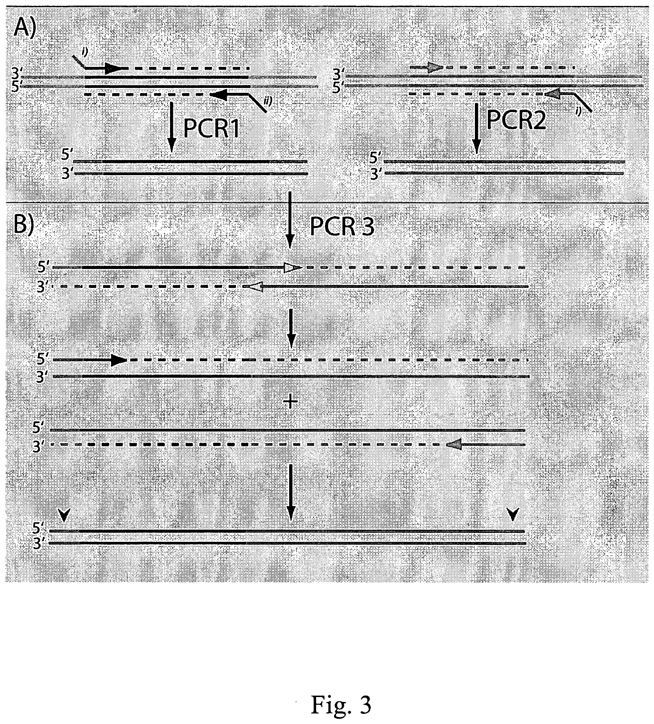

Figure 3 shows the principle of the PCR-based seamless cloning strategy: Initially two PCR reactions are performed using primers containing tails either complementary to the recipient vector and containing a restriction enzyme recognition site (indicated with i), or complementary to the other genetic element in the junction providing an overlapping region of the two PCR products to be fused (indicated with ii; Panel A). In a third PCR reaction, the 3' ends of the first PCR products initially function as "primers" before amplification takes place. In the resulting PCR product the two genetic elements (dark and light grey) are fused, and restriction enzyme sites (arrow heads) are introduced on each end to be used for the cloning of the fragment into the recipient vector (Panel B).

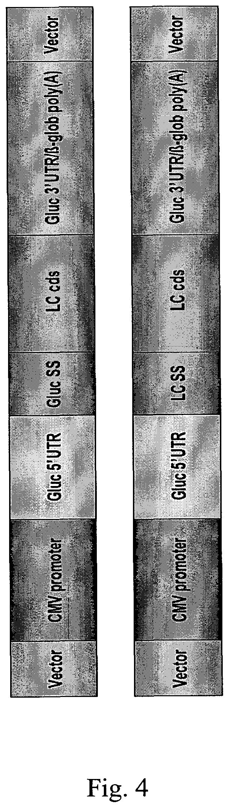

Figure 4 shows schematic maps of two expression cassettes encoding the cds of a human IgG light chain flanked by the indicated genetic elements.

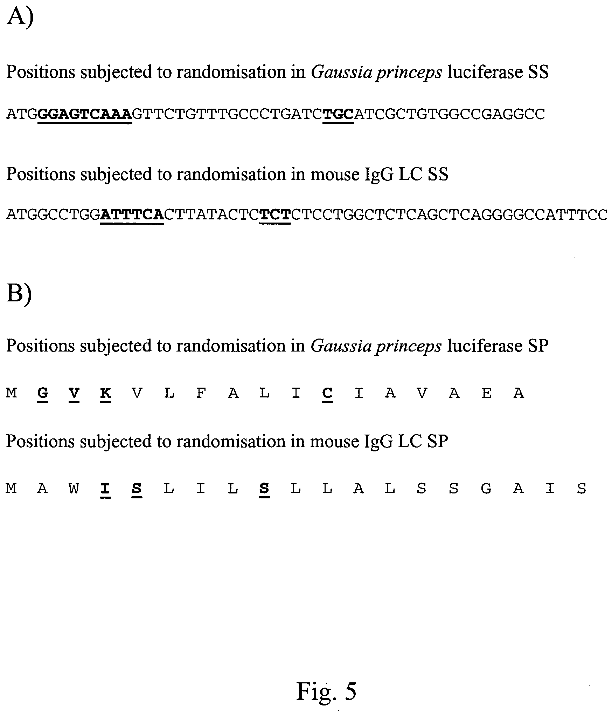

Figure 5 shows the DNA sequences of the SSs of *Gaussia princeps* luciferase and a mouse IgG light chain, respectively, with the positions subjected to randomisation underlined (Panel A) and the derived SP sequences where the respective positions are indicated at which the AAs will be randomised as a consequence of coding SS sequence randomisation (Panel B).

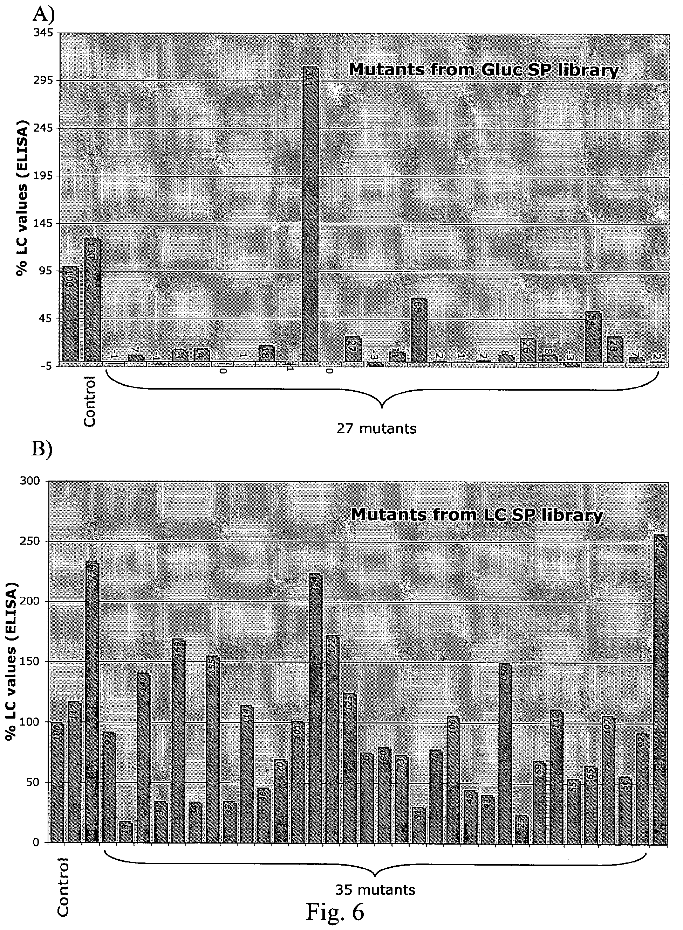

Figure 6 shows the ELISA analysis of human IgG light chain levels in medium samples from CHO cells transiently transfected with 27 mutant plasmids from either a *Gaussia* luciferase SP library (Panel A) or a mouse IgG light chain SP library (Panel B). In each case the LC value of the non-mutated construct (Control) was set to 100%.

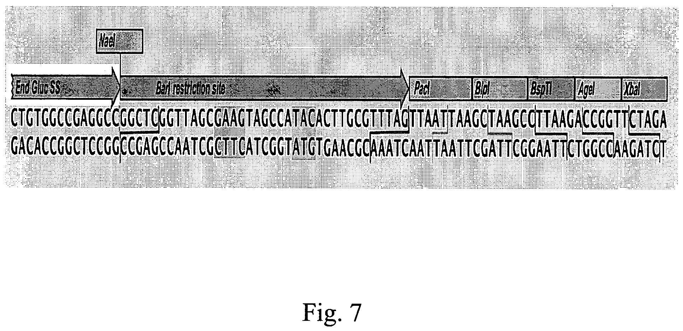

Figure 7 shows the cloning strategy for the insertion of a cds of any protein of interest into a pre-made library. The randomised genetic element, here exemplified by a randomised SS (only the 3' end being shown) is at its upstream end immediately followed by a multiple cloning site comprising the restriction enzyme recognition sites for a *Bar*I-type restriction enzyme cleaving away from its recognition site and for a series of rarely cutting enzymes, here exemplified by *Pac*I*, Blp*I, *Age*I and *Xba*I*.* Further, in the example shown here, where the randomised SS is a Glue SS, an enzyme (*Nae*I) cuts exactly at the junction between the Glue SS sequence and the sequence of the multiple cloning site. The cleavage sites (thin lines) of all enzymes and the recognition site of *Bar*I (shaded) are indicated. The genetic element-multiple cloning site unit is part of a preliminary vector. In order to incorporate a cds encoding a protein of interest into the vector, the vector will be cut with the *Bar*I-type enzyme and a suitable second enzyme the recognition site of which is located within the multiple cloning site downstream of *Bar*I*.* The cds, immediately followed by a 5'UTR and a polyadenylation signal, is provided by a PCR fragment which is designed such that it starts blunt-ended with the first codon of the cds and ends with a recognition site compatible with the one downstream of *Bar*I and used to open the preliminary vector. This site which must only be present once in the PCR fragment will be introduced into the fragment with the downstream PCR primer and located downstream of the polyadenylation signal. After cleaving the PCR fragment with the corresponding enzyme the fragment can be inserted into the pre-made library through blunt end-sticky end ligation thus ensuring a seamless connection between the randomised SS and the cds. Since *Bar*I-type enzymes generate sticky rather than blunt ends, treatment of the opened vector with a 3'->5' exonuclease has to precede ligation to remove the 3' overhang generated. In cases where there is a recognition site for a restriction enzyme present at the junction of randomised element and multiple cloning site not overlapping any randomised positions in the element and being cut by the enzyme such that a blunt-end is generated exactly at the element's upstream end (as by *Nae*I here), this enzyme can be used instead of a *Bar*I-type enzyme and the site for the latter omitted.

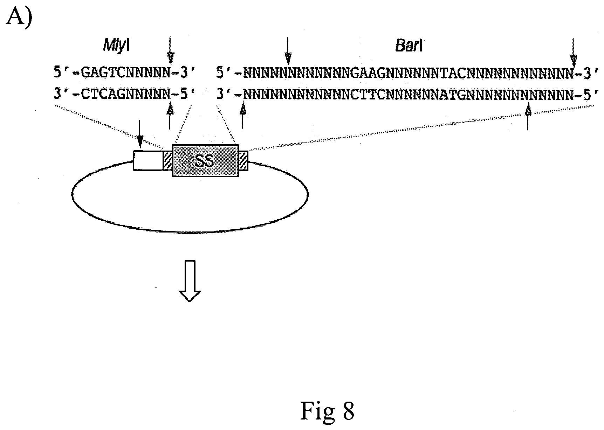

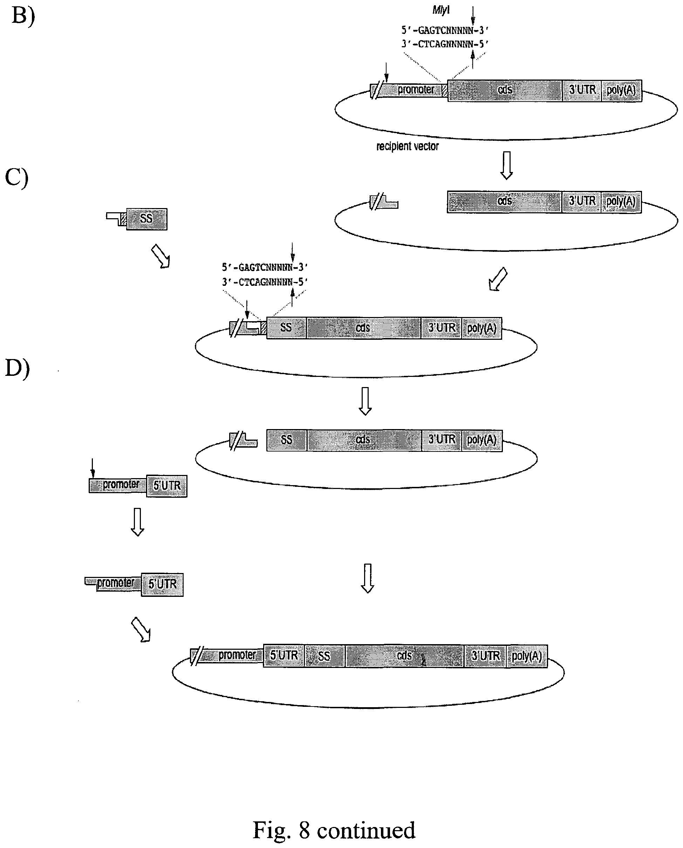

Figure 8 shows the cloning strategy for the insertion of a randomised genetic element from a pre-made library into a recipient vector harbouring the cds of a protein of interest. The randomised genetic element, here exemplified by a randomised SS located on a preliminary vector, is flanked by the recognition sites of a *Mly*I-type and a *Bar*I-type restriction enzyme, respectively, cleaving away (light arrows) from their recognition sites (specified nucleotides) (Panel A). They will enable the seamless connection between the randomised SS and the cds, being located on the recipient vector and immediately upstream preceded by a *Mly*I-type restriction enzyme recognition site (Panel B). The recipient vector will be cleaved with *Mly*I and a restriction enzyme cutting the vector only once and its recognition site being located upstream of *Mly*I, e.g. in the promoter (dark arrow), and combined with the randomised element, previously cut with *Bar*I and an enzyme with a recognition site compatible with the one upstream of the *Mly*I site in the recipient vector and used to open the vector (Panel C). The resulting construct is then re-cut with *Mly*I, the recognition site of which has been introduced with the randomised SS element in the previous step, and the enzyme cutting upstream of *Mly*I, and then seamlessly fused with the 5'UTR being provided as part of a PCR fragment designed such that it also replaces the part of the recipient vector lost during cloning (here the 3' part of the promoter) and such that it can be inserted into the recipient vector through sticky-end/blunt-end cloning (Panel D). Since *Bar*I-type enzymes generate sticky rather than blunt ends, the 3' overhangs they generate have to be removed with a 3'->5' exonuclease prior to blunt-end cloning. In case no suitable *Mly*I-type enzyme is available, it can be replaced with a *Bar*I-type enzyme of which there are many more available on the market. This merely requires additional 3'->5' exonuclease treatment steps to remove 3'-overhangs rather than generating blunt ends directly.

### DETAILED DESCRIPTION OF EMBODIMENTS OF THE INVENTION

### Definitions

Unless defined otherwise, all technical and scientific terms used herein have the same meaning as commonly understood by those ordinarily skilled in the art to which the invention belongs.

In the context of the present application and invention the following definitions apply:
The terms "modified/mutated" are used interchangeably within the text.
The term "protein of interest" is intended to mean any protein encoded by one or more genes, and of which there is a need for obtaining an increased quantity for specific purposes and which is to be produced in a recombinant manner by cultivated eukaryotic cells.

The term "coding sequence (cds)" is intended to mean a nucleotide sequence which begins with a start codon or the codon encoding the first amino acid of a mature protein and ends with a stop codon.

The terms "signal peptide (SP)" and "signal sequence (SS)" are intended to mean an N-terminal polypeptide targeting a protein for translocation across the endoplasmic reticulum membrane in eukaryotic cells and cleaved off during the translocation process, and the nucleotide sequence which codes for this polypeptide, respectively. Signal peptides may also be called targeting or localisation signals, signal or leader sequences or transit or leader peptides in the literature.

The term "5' untranslated region (5'UTR)" is intended to mean the nucleotide sequence in a mature mRNA located immediately upstream of any cds and not translated into protein. It extends from the transcription initiation site to just before the beginning of a cds.

The term "3' untranslated region (3'UTR)" is intended to mean the nucleotide sequence in a mature mRNA located immediately downstream of any cds and not translated into protein. It extends from the first nucleotide after the stop codon of any cds to just before the poly(A) tail of the mRNA.

The term "genetic element(s)" is intended to mean an mRNA element as well as any other nucleotide sequence involved in transcriptional and/or translational regulation of a gene, including but not limited to SS, 5'UTR, 3'UTR, enhancer, promoter, intron, polyadenylation signal and chromatin control elements such as MAR, UCOE and STAR, and any derivatives thereof.

The term "genetic element variant(s)" is intended to mean any genetic element which differs from the parental genetic element by one or more nucleotides and which has been generated by randomisation of said parental genetic element by using a mutagenic primer.

The term "randomised genetic element(s)" is intended to mean a pool of genetic elements being derived from one genetic element subjected to nucleotide randomisation at specific position(s).

The term "seamless cloning" is intended to mean a cloning method, such as PCR-based cloning, that results in the exact assembly of different genetic elements without incorporation of any linker DNA sequences (e.g. restriction sites) at the junctions between the different elements.

The term "secretion cassette" is intended to mean a nucleotide sequence containing the cds(s) of a protein of interest as well as at least the following genetic elements: a specific promoter, a specific 5'UTR, a specific SS, a specific 3'UTR and a specific polyadenylation signal.

The term "vector" is intended to mean a nucleotide sequence, usually being a circular double stranded DNA, having the ability to multiply independently of chromosomal DNA into numbers of copies in a host cell and may also integrate into the genome of the host cell. Furthermore, the vector is stably maintained and propagated in the host by making use of a selectable marker encoded by the vector. The vector may be a bacteriophage, a plasmid, a phagemid, an episomal vector, a viral vector, a plant transformation vector, an insect vector, or a yeast artificial chromosome.

The term "preliminary vector" is intended to mean a vector containing a specific genetic element, the nucleotide sequence of which is to be randomised at specific pre-defined positions, and equipped with special restriction enzyme recognition sites enabling the exact excision of said genetic element and its seamless insertion into any recipient vector containing the cds(s) of a protein of interest.

The term "final vector" is intended to mean a vector containing a specific genetic element(s), the nucleotide sequence(s) of which is/are to be randomised at specific pre-defined positions, and the cds(s) of a protein(s) of interest.

The term "recipient vector" is intended to mean any vector into which a DNA fragment is inserted by recombinant DNA technology.

The term "clonal cell line" is intended to mean the derivation of a cell line arising from a single cell.

The term "rational" is intended to mean based on reasoning and is used in this invention with respect to limiting random mutagenesis to specific positions within a genetic element according to practical experience and/or theoretical considerations.

The term "random" is intended to mean a process without order and is used in this invention with respect to the insertion of any of the four nucleotides at a specific position within a genetic element in an unbiased manner and with respect to selecting a certain number of samples from a pool in an unbiased manner.

The term "mutagenic primer" is intended to mean a synthetic oligonucleotide containing either a specific nucleotide(s) or any of the four nucleotides introduced at a defined position(s) and in this invention designed to cause incorporation of a mutation(s) at a specific position(s) in a genetic element.

The term "library" is intended to mean a pool of vector variants containing all variants of a specific genetic element generated by randomisation of its nucleotide sequence at specific pre-defined positions.

The term "pre-made library" is intended to mean a library generated with a preliminary vector.

The term "final library" is intended to mean a library generated with a final vector.

The terms "tailored" and "tailor-made" are intended to mean adjusted to specific needs and are used in this invention to describe libraries which are particularly efficient with respect to mediating increasd production yields of specific proteins/protein classes.

### The method of the invention and the rational library

The inventors have found that AAs at three specific positions (4, 5 and 13) in a chosen SP (derived from the Oik1 gene in the marine organism *Oikopleura dioica*) proved to be of particular importance in determining the efficiency by which the SP operates. A series of Oik1 SP mutants were generated, fused to the *Gaussia princeps* luciferase cds (reporter gene) and transfected into CHO cells. Large differences in luciferase activity were observed, ranging from 0 to almost 250% with respect to the activity achieved with pOik1 wild type SP. Exchange of leucine (position 4), serine (position 5), histidine (position 13) in the wild-type Oik1 SP with arginine, glycine, valine or arginine, threonine, tryptophan or lysine, valine, alanine in the respective positions led to decreased amounts of product, whereas the combinations valine, leucine, leucine or serine, phenylalanine, leucine resulted in increased amounts.

Identifying critical positions in any genetic element is a major challenge. Based on the inventor's knowledge gathered from a broad series of experimental data, visualisation of the SP patterns using hydropathy plots (Stern *et al.* 2007) as well as using bioinformatic tools we are now to a certain extent able to target such positions. Although the hallmark of an SP is a core hydrophobic region, we found that its hydropathic score is not a valid measure for the prediction of SP efficiency. Therefore it is imperative to achieve the highest diversity possible with respect to the different residues in each position of the element chosen to be randomised. To generate such high-quality libraries containing all variants without any bias for certain residue combinations and their application is the basis of our approach described here. Its most important aspect is the fact that it will enable "tailored" solutions. Defining the best elements with the best residue combinations for any protein to be produced goes far beyond the currently available "one-for-all" solutions where in a given production platform the same vector construct, though optimised for high expression, is applied to all proteins of interest. Only with such an approach can one realistically hope to reach the biological limit of production of a recombinant protein in a given expression system.

In one embodiment the invention provides a method for the increased production of a recombinant protein of interest in a eukaryotic cell, said method comprising
(i) providing a eukaryotic cell pool containing a final rational library of up to 4¹⁸ different vector variants containing randomised variants of a signal sequence and a gene encoding the recombinant protein of interest, said eukaryotic cell pool being obtained by a method comprising
   (a) providing said signal sequence and defining at most 18 nucleotide residues coding for at most 6 amino acid residues at specific positions in said signal sequence to be randomised; and
   (b) amplifying said signal sequence as part of a double stranded DNA plasmid, wherein said plasmid may be a preliminary vector, or a final vector containing a gene encoding the recombinant protein of interest, and subjecting said signal sequence to randomisation at said specified positions thereby generating a pool of randomised variants of said signal sequence as part of a vector; and (ci) amplifying said pool of randomised variants of said signal sequence being part of a preliminary vector, thereby generating a pre-made rational library, and introducing said pool of randomised variants of said signal sequence being part of the pre-made rational library into a recipient vector containing a gene encoding the recombinant protein in a seamless manner, thereby generating the final rational library, or (cii) amplifying said pool of randomised variants of said signal sequence being part of a preliminary vector, thereby generating a pre-made rational library, and introducing a gene encoding the recombinant protein into the pre-made rational library in a seamless manner, thereby generating the final rational library, or
   (ciii) amplifying said pool of randomised variants of said signal sequence being part of a final vector, thereby generating the final rational library; and
   (d) transforming said final rational library into eukaryotic cells thereby obtaining said eukaryotic cell pool; and
(ii) culturing said cell pool under conditions suitable for the production of said recombinant protein of interest; and
(iii) identifying within said cell pool a cell clone that produces the recombinant protein of interest at a higher level than that mediated by the unmodified signal sequence.

In certain embodiments a rational library is created by randomising 6, 7, 8, 9, 10, 11 or 12 nucleotides, coding for 2, 3 or 4 AA residues, thus said rational library may comprise from 4⁶=4096 up to 4¹² ≈ 1.7x10⁷ different vectors.

SPs show a remarkable level of divergence in AA composition, in fact the only unifying property shared by all SPs seems to be a stretch of at least 6 hydrophobic residues. The tolerability of divergent AA compositions is illustrated by the observation that up to 20% of all random 20-residue sequences can function as secretion signals in yeast. Despite the absence of a consensus sequence and a defined length, three distinct regions can be recognised in most SPs: First comes an amino-terminal 2-5 residue long positively charged region (n-region), followed by a 7-15 residue long hydrophobic core (h-region) and finally a 3-7 residue long polar carboxy-terminal region (c-region) containing the cleavage site recognised by a membrane bound signal peptidase. Positions -1 and -3, with respect to the cleavage site (0), are particularly important for specifying the cleavage site.

It was recognised early on that not all SPs are functionally equivalent (Knappskog *et al.* 2007, Stern *et al.* 2007, Tröße *et al.* 2007, Zhang *et al.* 2005). Many have reported that increased hydrophobicity is associated with enhanced translocation efficiency of SPs into the ER lumen. However, an upper limit for total hydrophobicity of SPs in mammalian cells may exist; one group created mutants with different degrees of hydrophobicity, and surprisingly the most hydrophobic SP was significantly less efficient in mediating translocation than less hydrophobic counterparts. Biophysical studies of SPs have demonstrated that functional SPs show a clear tendency toward stable α-helix formation in the hydrophobic core, to have high affinities for lipids (Jones *et al.* 1990). Position-dependent effects on hydrophobic core modifications on both translocation efficiency and SP cleavage indicate that the h-region has important structural properties in addition to hydrophobicity alone (Cioffi *et al.* 1989). Systematic introduction of α-helix breaking prolines in the h-region showed position-dependent inhibitory effects on glycoprotein C translocation, indicating functional asymmetry in the hydrophobic core of the SP (Ryan and Edwards 1995).

The charge of the amino-terminal basic region also has been shown to have an effect on SP efficiency. An SP with marginal hydrophobicity in the h-region depends on a sufficient positive charge at the n-region for translocation to occur (Rusch *et al.* 2002). This dependence diminished when the stretch of hydrophobic residues was increased, indicating that the requirement of positive charge can be compensated for by a longer hydrophobic core (Hikita and Mizushima 1992). Separating the positive charged n-region from the h-region with more than four AAs abolished SP function, indicating that the positioning of these elements is crucial for promoting protein transport into the ER (Rusch *et al.* 2002). Introduction of negative residues in the n-region has a negative impact on translocation efficiency (Szczesna-Skorupa and Kemper 1989; Izard *et al.* 1996). The impact of one negative residue in the n-region can be rescued by a highly hydrophobic h-region, but if as many as 3 negative residues are incorporated then the positive effect of this core will be severely affected (Szczesna-Skorupa and Kemper 1989). Growing evidence indicates that SPs contain more information than just simple "tags" for targeting to the ER lumen (Hegde and Bernstein 1998; Martoglio and Dobberstein 1998).

Said SS may be selected from the group consisting of SSs from human, rodent, *Gaussia princeps, Metridia longa* and *Oikopleura dioica*, and mutants derived thereof. Examples of nucleotide sequences that contain SSs are SEQ ID NO:1, 2, 3, 4, 5, 6, 7, 8, 9, 10, 11 or 12. Specific examples include SEQ ID NO:1, 2, 3, 4, 5 or 6. The SS may be derived from *Gaussia princeps.*

One example is the method as described above with which a rational library is created to randomise 9 or 12 nucleotides coding for 3 or 4 AAs, thus randomising 3 or 4 AAs.

The rational library may be a pre-made library equipped with restriction enzyme recognition sites enabling the seamless insertion of the randomised genetic element within said pre-made library into any recipient vector encoding a protein of interest thus generating a final library or the insertion of any cds of a protein of interest into the pre-made library and thus generating a final library. The restriction enzyme recognition sites to be used may vary depending on the sequence of the recipient vector or cds, respectively. For the insertion of the randomised genetic element into a recipient vector the sites must be present only once in the recipient vector and for the insertion of a cds into the pre-made library they must not cut the cds. To choose such sites is well-known for a person skilled in the art.

The vector used in the library may be an episomal vector suitable due to the fact that it replicates extrachromosomally rather than by integrating into the genome of the host cell. The production level of the recombinant protein thus would not be affected by the integration site which could "camouflage" the effect of the individual vector variants in the library by promoting increased or decreased levels of transcription depending on the status of the chromatin. The library of use in the invention may be generated directly in an episomal vector, or be moved into an episomal vector before being transfected into a cell line such as a mammalian cell line.

The eukaryotic cells into which the libraries are transferred may be selected from the group consisting of cells derived from animal, plant, fungi and yeast systems, such that the animal cell may be a mammalian or insect cell. Eukaryotic cells have the ability to perform glycosylation. However, different eukaryotic cells may give rise to different glycosylation patterns and some of the eukaryotic cells may give rise to a pattern that is different to that of the native protein to be produced. In that case, for that particular protein, another eukaryotic cell line would have to be used. Examples of different eukaryotic cells that may be used in the invented method are murine lymphoid cell lines, baby hamster kidney cell lines, human embryo kidney cell lines, human retina-derived cell lines, Chinese hamster ovary cell lines. Other examples are that said mammalian cell is selected from the group consisting of primate-, monkey- and rodent-derived cells. Other examples are that said primate cell is of *Homo sapiens* or *Pan troglodytes* origin, said monkey cell is of *Cercopithecus aethiops* origin, and said rodent cell is of *Cricetulus griseus*, *Mesocricetus auratus*, *Rattus norvegicus*, *Oryctolagus cuniculus* or *Mus musculus* origin. Other examples are that said mammalian cell belongs to any of the cell line families CHO, SP2/0, NS0, 293, myeloma, NOS, COS, BHK, HeLa and PER.C6, and derivatives thereof.

In one embodiment the genetic element variants may be generated either by gene synthesis where random nucleotides are incorporated at specific positions or by Thermal Cycling utilising a mutagenic primer. The mutagenic primer used in the Thermal Cycling reaction comprises all randomised nucleotides at all specified positions and has a length between 60 and 100 nucleotides, a total TM from 70 to 85 °C and similar TMs with values from 55 to 70 °C at both non-mutated ends flanking the mutated region.

The production level of the proteins of interest may be determined by the use of an enzyme-linked immunosorbent assay (ELISA), a bioluminescence assay, Western blot analysis, Protein A HPLC, or by any other suitable method as disclosed e.g. in the above mentioned manuals by Sambrook *et al.* and Ausubel *et al.*

Also described is a method to generate rational libraries comprising genetic elements which are involved in the expression of a gene and devised to increase the production yield of the encoded protein, comprising the steps of;
a) providing a genetic element to be optimised for expression capacity and defining at most 18 nucleotide residues, either non-coding or coding for at most 6 amino acid residues at specific positions in said genetic element to be randomised,
b) amplifying said genetic element, said genetic element being part of a double stranded DNA plasmid being a preliminary vector or a final vector and subjecting said genetic element to randomisation and generating a pool of genetic element variants,
c) amplifying said pool of genetic element variants being part either of a preliminary vector, thus generating a pre-made library or being part of a final vector, thus generating a final library, or
d) introducing said pool of genetic element variants being part of a preliminary vector into a recipient vector in a seamless manner, thus generating a final library,
e) transforming said final library into eukaryotic cells and
f) obtaining a eukaryotic cell pool containing a rational library comprising up to 4¹⁸ ≈ 6.9x10¹⁰ different vector variants.

In the above described method said genetic element may be selected from the group consisting of SS, 5'UTR, 3'UTR, enhancer, promoter, intron, polyadenylation signal and chromatin control elements or other genetic elements that might be involved in transcriptional and/or translational regulation of the encoded protein or in mRNA stability, wherein the genetic element is randomised at the level of the nucleotide sequence which in cases where the genetic element is a cds, will give rise to a randomisation at the AA level. Examples of chromatin control elements are selected from the group consisting of MAR, UCOE and STAR.

Also described herein is a method to identify a clonal cell line harbouring a vector variant where said clonal cell line produces the highest amount of the protein of interest, comprising the steps of
a) generating the genetic element variants in a vector containing the gene encoding the protein of interest or incorporating said genetic element variants from a pre-made library into a vector containing the gene encoding the protein of interest,
b) screening for the cell clone that produces the protein of interest to the highest level and
c) obtaining a clonal cell line from the rational library that mediates the highest level of production of the encoded protein.

The method may include a step, wherein said screening is performed by flow cytometry and/or cell sorting.

Also described herein is a rational library based on a vector containing different genetic elements which have been seamlessly cloned, said rational library containing up to 7x10¹⁰ different vector variants wherein each variant contains at most 18 changed nucleotides, either non-coding or coding for at most 6 amino acid residues, at specific positions in one of the genetic elements and wherein each vector variant mediates a different expression level of the encoded protein of interest as compared to the non-modified vector. Said rational library may be obtained by the method disclosed above as well as using the steps disclosed in the examples below. The rational library may contain vectors as defined above, wherein said vectors may contain SS, 5'UTR, 3'UTR, enhancer, promoter, such as the human cytomegalovirus major immediate-early promoter/enhancer (hCMV promoter), intron, polyadenylation signal and chromatin control elements such as MAR, UCOE and STAR. The vectors may also contain origin of replication, restriction enzyme recognition sites as well as one or more selection marker genes. The vector may also contain one or more genes to be expressed by said vector. Said gene of interest may be cloned into said vector in a manner well-known for a person skilled in the art, such as by the use of a suitable method disclosed in the well-known manuals Sambrook J et al. (Molecular Cloning: A Laboratory Manual (Third Edition), Cold Spring Harbor Laboratory Press, Cold Spring Harbor, N.Y., 2001) and Ausubel FM et al. (Current Protocols in Molecular Biology, Wiley InterScience, 2010). The vector may then be introduced into a eukaryotic host cell line and the cells containing the vector may be selected by cultivating the cells in a medium containing a selection agent, such as hygromycin B phosphotransferase, or puromycin, depending on the particular selection marker present in the vector used.

Also described is the use of the method as described above for the increased production of recombinant proteins in a eukaryotic cell. The rational libraries disclosed above may be used for but are not limited to the use for biologics, biosimilars, industrial proteins and proteins for research.

### EXAMPLES

The following examples are intended to illustrate but not to limit the invention in any manner, shape, or form, either explicitly or implicitly.

### Materials

The chemicals, enzymes, media and solutions used for the creation, verification and application of the libraries are commonly used and well known for a person skilled in the art of molecular and cell biology; they are available from a number of companies including Amersham, Invitrogen, Stratagene, Sigma, Merck, Fluka, Medicago, Promega, Fermentas and Qiagen, many of them being provided in kits.

### Methods

Unless indicated otherwise, the methods used in this invention including Polymerase Chain Reaction (PCR), restriction enzyme cloning, DNA purification, bacterial and eukaryotic cell cultivation, transformation, transfection, Western blotting and Enzyme-Linked Immuno Sorbent Assay (ELISA), were performed in a standard manner well known for a person skilled in the art of molecular and cell biology, and such as described in the following manuals: Sambrook J et al. (Molecular Cloning: A Laboratory Manual (Third Edition), Cold Spring Harbor Laboratory Press, Cold Spring Harbor, N.Y., 2001) and Ausubel FM et al. (Current Protocols in Molecular Biology, Wiley InterScience, 2010).

### EXAMPLE 1

Identification of key positions in any SP, providing the basis to generate rational SP-based libraries

It has been shown previously that the choice of SP is crucial when constructing vectors with the aim of increasing the production level of a recombinant protein. *Gaussia princeps* luciferase SP (Gluc SP) has proved to be a far better SP than a whole series of other SPs tested in an expression system (transfected CHO cells) using *Gaussia* luciferase as a reporter protein (Knappskog *et al.* 2007; Stern *et al.* 2007).

Among the SPs tested were seven derived from the marine organism *Oikopleura dioica.* Only one of these, namely the SP of the oikosin 1 protein (Oik1 SP) gave substantial activity, amounting to 45% of that generated when the Gluc SP was used (Tröße *et al.* 2007). From hydropathy plots of the two SPs (Figure 1) it is evident that the h-region of each SP was of appropriate length and in both cases consisted only of hydrophobic AAs. Three major differences were observed: (1) Gluc SP has a very hydrophilic AA (lysine) in position 4 while Oik1 SP has a hydrophobic AA (leucine) in this position, (2) Gluc SP has a hydrophobic AA (valine) in position 5 while Oik1 SP has a hydrophilic AA (serine) in this position, (3) Gluc SP has a hydrophobic AA (valine) in position 13 while Oik1 SP has a hydrophilic AA (histidine) in this position. It was thus considered that AA positions 4 and 5, and 13, represent potential sites for investigating whether or not specific mutation could improve the effectiveness of the Oik1 SP.

The template for all plasmids used in a pilot study was a derivative of pTRE2hyg (Clontech) containing a secretion cassette composed of the 5'UTR, the cds and the 3'UTR of *Gaussia* luciferase cDNA (GenBank accession no. AY015993) and the Oik1 SP cds (SEQ ID NO:9) immediately preceding the luciferase cds. For mutagenesis of the Oik1 SP at positions 4, 5 and 13 the QuikChange Site-Directed Mutagenesis Kit from Stratagene was used (according to the manufacturer's recommendations) and synthetic oligonucleotide primers containing the desired mutations.

Twenty constructs encoding Oik1 SP mutants were generated (termed p---, with the "-" sign depicting AA positions 4, 5 or 13 in the Oik1 SP and being replaced by the single-letter code for AAs if the AA differs from that in the wild-type Oik1 SP) and the constructs as well as the reference construct encoding the wild-type Oik1 SP (termed pOik1) transiently transfected into CHO cells using the MATRa technology by IBA (according to the manufacturer's recommendations). Medium samples were collected after 30 h and the luciferase activity was measured in the medium following to the method described below. The results are shown in Table 1. In order to compare RLU values from different measurements, the value obtained with pOik1 has been set to 100% and the other values have been adapted accordingly.

It can be seen from Panel A in Table 1 that the combination of AAs in positions 4 and 5 had a major impact on the level of luciferase production. The plasmid encoding the Oik1 SP mutant where leucine was switched to arginine and serine to isoleucine (pRI-) was by far the most efficient of this series, giving rise to 125% more activity than plasmids pFS- and pVQ-, and almost 90% more activity than pOik1. The switch of AA in position 13 (histidine in the wild-type Oik1 SP) had an even greater impact on luciferase activity levels (Panel B). The plasmid encoding the Oik1 SP mutant with phenylalanine in this position (p--F) gave rise to more than 170% more luciferase than p--A and almost 150% more than pOik1. Mutations in all three positions, namely 4, 5 and 13 (Panel C), also resulted in large differences in the levels of luciferase produced. Three mutant plasmids gave lower levels of luciferase than pOik1 while the other two were very effective in producing luciferase.

From these results it is evident that the ability to predict which AA is suited for a specific position in the SP is virtually impossible. For example, the pKV-mutant (Table 1, Panel A) in the Oik1 SP contains the same AAs in positions 4 and 5 as is found in the Gluc SP (see Figure 1) but this result in a 30% decrease in luciferase production when compared to the Oik1 SP. This suggests that a doublet of AAs in a specific position that functions well in one SP does not necessarily function as well in another SP, indicating the importance of other AAs "downstream" in the SP.

An interesting correlation between the improvement in efficiency of the Oik1 wild type SP by mutation and the use of bioinformatics (SignalP 3.0 Server - http://www.cbs.dtu.dk/services/SignalP/) was demonstrated. As seen in Table 1, Panel B, mutation of histidine (wild type) in position 13 to phenylalanine causes a major increase in luciferase production. Comparison of the two S-score plots (Figure 2, upper panel) shows that the mutant SP gives an improved plot. It appears, however, that the S-plot is improved "upstream" of the mutated AA in position 13, and in this case not "downstream". Interestingly, the S-score plot was not improved such that it was coincident with the site of mutation. A further example is given in the lower panel of Figure 2 (data from Table 1, Panel C - pSFL mutant). In this case mutations at sites 4 and 5, and 13, improved the S-plot in a region of the SP between the mutation sites.

The results shown here demonstrate that the choice of making mutations in positions 4 and 5, and 13 of the Oik1 SP, based on a study of the hydropathy plots, was a correct strategy to provide a basis for improving the performance of the Oik1 wild type SP. It is envisioned that in the future it will be fruitful to couple the use of hydropathy plots with the bioinformatics approach in the identification of positions in SPs that can have a high impact on recombinant protein yields.

### Gaussia Luciferase activity assay

Luciferase activity in the medium sample was measured as the amount of photons released when the sample was mixed with coelentrazine (Promega) in a Chameleon multilabel counter (Hidex Oy). Two samples for every cell line transfected with a specific construct were removed from the -80°C freezer, and thawed on ice. To find the optimal dilution, a dilution assay with *Renilla* buffer (Promega) was performed by measuring dilutions of the samples in the luminometer. When a linear area was found, a suitable dilution was chosen for the real measurements of the samples. Ten µl of the diluted samples were added to each of 2 wells in a 96-well plate placed on ice. The plate was put into the lunimometer and to each well was added 150 µl of standardized coelenterazine solution (*A*₂₆₇ ≈ 0.400) by the dispenser. The Relative Light Units (RLUs) data obtained from the luminometer were corrected for dilutions made and for the number of cells present in the well the sample was taken from (determined with the Nucleocounter from Chemometec).

**Table 1**

| Levels of *Gaussia* luciferase activity in the medium from CHO cells transfected with plasmids containing various mutants of the Oik1 SP: positions 4 and 5 (Panel A), position 13 (Panel B) and positions 4, 5 and 13 (Panel C). | | | | | | | | | |
|---|---|---|---|---|---|---|---|---|---|
| A) | | | | | | | | | |
| Plasmid^{a)} | pOik1(LS-) | pEH- | pFS- | pVQ- | pKV- | PQY- | pVP- | pLP- | pRI- |
| Luciferase activity in medium^{b)} | 100 | 0 | 63 | 63 | 70 | 89 | 126 | 142 | 188 |

| B) | | | | | | | | | |
|---|---|---|---|---|---|---|---|---|---|
| Plasmid^{a)} | pOik1 (--H) | p--A | p--R | p--P | p--I | p--M | p--C | p--F | |
| Luciferase activity in medium^{b)} | 100 | 70 | 82 | 89 | 185 | 191 | 224 | 246 | |

| C) | | | | | | | | | |
|---|---|---|---|---|---|---|---|---|---|
| Plasmid^{a)} | pOik1 (LSH) | pRGV | pRTW | pKVA | pVLL | pSFL | | | |
| Luciferase activity in medium^{b)} | 100 | 12 | 74 | 77 | 201 | 222 | | | |

| | | | | | | | | | |
|---|---|---|---|---|---|---|---|---|---|
| ^{a)} For the plasmid encoding the wild-type Oik1 SP, pOik1, the AAs at positions 4 and 5 and/or position 13 are given in brackets in consecutive order. The same order is included in the name of the plasmids encoding the mutant Oik1 SPs, p---, with the amino acid(s) differing from the wild-type Oik1 AA(s) specified. ^{b)} Luciferase activity measured in the medium obtained from 3 independent transfections, given in percent with respect to the value obtained with pOik1 set to 100%. | | | | | | | | | |

### EXAMPLE 2

### Library generation

### Design of expression cassette

The model protein chosen is a human IgG light chain (LC) derived from GenBank accession no. AB064226. Its cDNA cds without native SS was at the 5' end fused either to the codon optimised SS from *Gaussia princeps* luciferase (SEQ ID NO:6) or the SS from a mouse IgG LC (SEQ ID NO:12) and at the 3' end to the 3'UTR from *Gaussia princeps* luciferase. The respective SSs were at their 5'ends fused to the 5'UTR from *Gaussia princeps* luciferase, extended at its 5'end with the sequence 5'-ATTCAGACAACTGAATCCAAAAGGAAA-3'. The respective 5'UTR-SS-cds-3'UTR units were inserted between the human cytomegalovirus major immediate-early enhancer/promoter (derived from GenBank accession no. NC_006273) at the 5' end and the rabbit beta-globin polyadenylation signal (derived from GenBank accession no. RABBGLOB) at the 3' end. Assembly of the various sequences was performed by seamless cloning. The method used is outlined in Figure 3 and the resulting expression cassettes shown in Figure 4.

### Vector for library construction

The vector used is a derivative of pcDNA3.1(+) (Invitrogen). The respective expression cassettes equipped with appropriate restriction enzyme recognition sites at their 5' and 3' ends were inserted into the vector by restriction enzyme cloning.

### Randomisation of specific SS positions

For randomisation the QuikChange Multi Site-Directed Mutagenesis Kit or the QuikChange Lightning Multi Site-Directed Mutagenesis Kit (Stratagene) were used. Although designed for the site-directed mutagenesis of plasmid DNA at different sites simultaneously and suitable for nucleotide randomisation, the following adaptations were required to make the kits suitable for library generation: (i) Since the positions within the SSs to be randomised were located rather close to each other, the incorporation of mutations had to be performed with one primer instead of several primers, as recommended by the manufacturer. In order to contain sufficiently long stretches with parental (non-mutated) sequences flanking the region with the mutations, the primers had to be longer than recommended (60 to 100 nucleotides instead of 25-45 nucleotides). They were designed such that their total TM was between 70 and 85 °C and the TMs of the flanking stretches between 55 and 70 °C and as similar as possible. (ii) The amount of QuikSolution (provided with the kit) in the Thermal Cycling reaction was increased from 3% to 4% and the reaction volume from 25 µl to 50 µl. (iii) The incubation time with *Dpn*I was prolonged from 1 h to 6 h.

The positions chosen for mutagenesis in the codon optimised SS from *Gaussia princeps* luciferase (Gluc SS) and the SS from mouse IgG LC (LC SS), respectively, are shown in Figure 5A. In Gluc SS four codons were degenerated, resulting in four randomised AAs and in LC SS three codons were degenerated, resulting in three randomised AAs (Figure 5B). The mutagenic primers used for Gluc SS and LC SS randomisation are as shown (SEQ ID NO:13 and 14).

### Library harvesting

Transformation of *E. coli* XL-10-Gold ultracompetent cells was performed by using 4 µl aliquots of DNA from the mutagenesis reaction for each transformation reaction. The whole volume of the transformation reaction was plated on Luria Bertani (LB) agar plates (diameter 15 cm) containing ampicillin (100 µg/ml) for plasmid selection, and the LB agar plates incubated o/n at 37°C. Subsequently, 10 ml LB medium was added to each LB agar plate and the colonies scraped off with a cell scraper and collected in two GSA bottles. Another 10 ml LB medium was added to each plate to rinse and collect any remaining bacteria on the plates. This colony mix was then directly subjected to plasmid DNA purification using the Qiagen Plasmid Mega Kit (Qiagen) according to the manufacturer's recommendation.

### EXAMPLE 3

Assessment and adjustment of critical parameters during library generation, to achieve high-quality libraries.

### Test transformation

Prior to harvesting the library (see Example 2), a test transformation was performed. This was done in order to assess the transformation efficiency (measured as cfu (colony forming units) per µg pUC18 control plasmid), the number of colonies formed per transformation using 4 µl aliquots of the mutagenesis reaction, as well as the mutagenesis efficiency, i.e. the number of mutants obtained per number of transformants. To assess the latter, approx. 50 single colonies were randomly selected, inoculated and incubated, the cultures were then subjected to plasmid DNA purification and the DNA sequence in the region of the SS was determined. When the parameters were satisfactory, i.e. high transformation and mutagenesis efficiencies and colony number achieved, the libraries were harvested.

### Parameter adjustment

The quality of a library is determined by two criteria, namely size and diversity. A library generated based on 9 randomised nucleotides (LC SS) or 12 randomised nucleotides (Gluc SS), respectively (see Figure 5A), would have to contain 4⁹ ≈ 2.6x10⁵ or 4¹² ≈ 1.7x10⁷ different vector variants, respectively, in order to be complete. Since the statistics of sampling such large pools of variants, however, ensures that every possible variant is not necessarily represented at all or is represented more than once, even when the underlying method of library construction is unbiased (i.e. all four bases occurring with the same frequencies at all randomised positions), the numbers are even higher. Using the GLUE programme (http://guinevere.otago.ac.nz/aef/STATS/index.html) developed by Patrick and Firth (2005), completeness of the respective libraries, based on a 95% probability, is calculated to require sizes of 4.0x10⁶ or 3.3x10⁸.

According to these numbers, with the Gluc SS and the LC SS libraries a completeness of approx. 10% was achieved by adjusting the following parameters:
a) Increase of transformation efficiency by avoiding any change in storage temperature (-80°C) of the *E.coli* XL-10-Gold ultracompetent cells. A storage box exposed to a higher temperature while taking out vials of cells for usage was not placed back and any remaining vials discarded. The transformation efficiency achieved was up to 1.2x10⁹.
b) Increase of mutagenesis efficiency by employing the QuikChange Lightning Multi Site-Directed Mutagenesis Kit instead of the QuikChange Multi Site-Directed Mutagenesis Kit from Stratagene. The former, newer kit contains an optimised enzyme blend and *Dpn*I restriction enzyme. This results in a more efficient mutant ssDNA synthesis and a more efficient removal of the parental DNA, which together clearly increases the mutated to non-mutated ratio. The mutagenesis efficiency achieved was up to 80%.
c) Reduction in bias towards any nucleotide by employing high-quality primers. It appeared that primer quality dropped with time and that an older primer, though properly stored, resulted in a drastic overrepresentation of Adenine residues at the mutated positions. Almost no nucleotide bias was observed when using a freshly synthesised mutagenic primer.
d) Reduction in bias towards any position to be mutated by increasing the annealing temperature in the Thermal Cycling reaction. Depending on the sequence of the primer, secondary structures may form and prevent certain positions located in hairpin loops from annealing. When raising the annealing temperature during Gluc SS mutant strand synthesis from 55°C to 65°C, any such potential structure in the corresponding primer (SEQ ID NO:13) apparently disappeared. Instead of obtaining a clear overrepresentation of mutants in positions 31-33 with a temperature of 55°C, no such bias was observer with a temperature of 65°C.
e) Increase of transformant numbers by performing several transformations in parallel. Here 10 (for LC SS) and 20 (for Gluc SS) transformations were performed and the colonies pooled, respectively.

### EXAMPLE 4

### Proof-of-concept of the library approach

In order to evaluate the potential of the library approach and establish proof-of-concept two SS/SP libraries were constructed, both containing the human IgG LC cds to which either the Gluc SP (Library 1), or the LC SP (Library 2) was fused (for details see Example 2). Using the approach described in Example 1, in Gluc SP positions 2, 3, 4 and 11 were identified as key positions, and in LC SP positions 4, 5 and 9 were identified as key positions. The libraries were generated accordingly. CHO cells transiently transfected with randomly selected mutants were grown and medium samples were collected 30 h after transfection. Three parallel experiments were performed and the IgG LC levels in the medium were measured either by ELISA and Western blot. The results obtained from the two methods were similar; in Figure 6 only results from ELISA analysis are shown.

From Library 1 a total of 27 distinct variants of the Gluc SPs were tested, and from Library 2 a total of 35 distinct variants of the LC SPs were examined. None of them contained stop codons or frame-shift mutations. The mutant AA sequences together with the N-terminal sequence of the LC protein were examined by using the SignalP server (http://www.cbs.dtu.dk/services/SignalP/) to ensure that they would function as SPs and also to predict the cleavage site.

In Library 1 a great variation in the expression levels of the mutants was seen, ranging from 0% to 311 % compared to the non-modified Gluc SP (Figure 6A). In many of the SP mutants LC secretion seemed to be totally abolished; only 15 of the mutants secreted LC at detectable levels. Analysis of the random clones from Library 2 also showed great variation, ranging from 18% to 259% compared to the native LC SP (set to 100%) (Figure 6B). In contrast to the Gluc SP all the LC SP mutants mediated LC secretion. In order to validate the results nine mutants from each library were selected and co-transfected in 3 individual parallel experiments together with a *Firefly* luciferase encoding plasmid. The LC levels in the medium were measured by ELISA and the *Firefly* activity in the extracts of the corresponding cells was determined with a bioluminescence assay (see below). This was performed in order to avoid variation caused by deviation in transfection efficiency. The results confirmed those obtained in the initial transient transfection experiments.

From these observations demonstrating the high degree of variation seen in the small subset of mutants analysed from the two libraries it is evident that such a library approach to improve protein production has a tremendous potential. The SP mutants had a major influence on the secretion levels of the human IgG LC protein. The variation in levels obtained was extremely marked. It was of interest to note that the mutant that gave the highest yield of LC was derived from Gluc SP and was not among the LC SP mutants. It is thus evident that the most effective SP for a given protein need not necessarily have to be derived from the same class of proteins. The results also clearly demonstrate the level of variability among individual SP mutants, showing that mutations of single AAs at pre-chosen sites based on theoretical considerations (see Example 1) have a major impact on yield. It will, though, seemingly be impossible to predict a "best-performing" SP based on AA sequence data alone.

The outcome of this study has three important aspects:
1) It provides the proof-of-concept for the rational library approach.
2) This approach can be continuously improved by relating the biological data back to the theoretical considerations based on hydropathy plots and bioinformatics outlined in Example 1 in order to obtain even more improved SPs providing yields approaching the biological limit.
3) Finally, the study demonstrates that aiming to tailor the library approach is a direction of high potential. For any protein of interest optimal SPs will be identifiable in this way.

### Firefly Luciferase Activity Assay

In order to normalise the IgG LC levels in each medium sample for varying transfection efficiencies, co-transfection with 0,5 µg *Firefly* luciferase encoding plasmid (together with 2 µg of the LC encoding plasmid to be analysed) was performed.

Extracts from the cells were thawed on ice and measured for *Firefly* luciferase activity using the Luciferase Assay from Promega. First a dilution series of one of the samples was performed in *Renilla* buffer to determine the linear range of light detection. Then all samples to be analysed were diluted to an appropriate concentration. The luciferase substrate was prepared according to the manufacturers recommendations. Twenty µl of each sample was loaded onto a 96-well plate and the plate then placed into a Chameleon multilabel counter (Hidex Oy). At room temperature 100 µl luciferase assay substrate was stepwise added to each well and the RLUs measured.

For normalisation of the ELISA absorbance values obtained for IgG LC, standard curves for both ELISA and the *Firefly* assay were made and the A₄₅₀ values divided by the RLU values.

### EXAMPLE 5

### The concept of pre-made libraries

Several steps during library generation have been optimised (see Example 3) and it will therefore be a relatively straightforward task to obtain rational libraries with maximal size and diversity. To reach 100% completeness will mainly be a matter of increasing the number of transformations performed in parallel. On the other hand, the quality of any *de novo* created library may vary and verification of size and diversity is time consuming. A "standardisation" would therefore be desirable, where pre-made, quality checked libraries, based on a variety of specific genetic elements randomised at critical positions, could be taken off-the-shelf and applied to any protein of interest. Such libraries, when being re-used repeatedly for various proteins, would, in addition, contribute to the understanding of which proteins or protein classes would benefit most from which type of library. This could considerably speed up and broaden the process of being able to generate "tailor-made libraries" for specific proteins/protein classes in the future.

The challenge of establishing this concept is to devise a cloning strategy for the pre-made libraries. The randomised genetic element has to be seamlessly fused with adjacent elements (i.e. the randomised SS with the 5'UTR and protein cds on either side, respectively), which cannot be performed using a PCR-based cloning approach, such as the one outlined in Figure 3, since the nucleotide overlap at the fragment junctions introduced with a primer would eliminate any diversity at the randomised positions. The solution is provided by special restriction enzymes which cleave away from their recognition sequence. Using such enzymes, both the insertion of a cds of any protein of interest into a pre-made library (Figure 7) as well as the insertion of a randomised genetic element from a pre-made library into a recipient vector harbouring the cds of a protein of interest (Figure 8) is feasible. The former approach opens the opportunity to generate the pre-made library in an optimised expression vector, thus adding the benefit of the library to the high transcription rate mediated by the vector. The latter approach allows for the integration of the randomised genetic element into any vector being part of a proprietary production platform. Many other applications are, of course, conceivable.

### REFERENCES

Cioffi, J.A., Allen, K.L., Lively, M.O. & Kemper, B. Parallel effects of signal peptide hydrophobic core modifications on co-translational translocation and post-translational cleavage by purified signal peptidase. J. Biol. Chem. 264 (1989) 15052-15058.
Eisenberg, D., Weiss, R.M., Terwillinger, T.C. & Wilcox, W. Hydrophobic moments in protein structure. Faraday Symp. Chem. Soc. 17 (1982) 109-120.
Hegde, R.S. & Bernstein, H.D. The surprising complexity of signal sequences. Trends. Biochem. Sci. 31 (1998) 563-571.
Hikita, C. & Mizushima, S. The requirement of a positive charge at the amino terminus can be compensated for by a longer central hydrophobic stretch in the functioning of signal peptides. J. Biol. Chem. 267 (1992) 12375-12379.
Izard, J.W., Rusch, S.L. & Kendall, D.A. The amino-terminal charge and core region hydrophobicity interdependently contribute to the function of signal sequences. J. Biol. Chem. 271(1996) 21579-21582.
Jones, J.D., McKnight, C.J. & Gierasch, LM. Biophysical studies of signal peptides: implications for signal sequence functions and the involvement of lipid in protein export. J. Bioenerg. Biomembr. 22 (1990) 213-232.
Knappskog, S., Ravneberg, H., Gjerdrum, C., Tröβe, C., Stern, B. & Pryme. I.F. The level of synthesis and secretion of Gaussia princeps luciferase in transfected CHO cells is heavily dependent on the choice of signal peptide. J. Biotechnol. 128 (2007) 705-715.
Martoglio, B. & Dobberstein, B. Signal peptide: more than just greasy peptides. Trends Cell Biol. 8 (1998) 410-415.
Patrick, W.M. & Firth, A.E. Strategies and computational tools for improving randomized protein libraries. Biomol. Eng. 22 (2005) 105-112.
Rusch, S.L., Mascolo, C.L., Kebir, M.O. & Kendall, D.A. Juxtaposition of signal-peptide charge and core region hydrophobicity is critical for functional signal peptides. Arch. Microbiol. 178 (2002) 306-310.
Ryan, P. & Edwards, C.O. Systematic introduction of proline in a eukaryotic signal sequence suggests asymmetry within the hydrophobic core. J. Biol. Chem. 270 (1995) 27876-27879.
Stern, B., Olsen, L.C., Tröβe, C., Ravneberg, H. & Pryme, I.F. Improving mammalian cell factories: The selection of signal peptide has a major impact on recombinant protein synthesis and secretion in mammalian cells. Trends in Cell & Molecular Biology 2 (2007) 1-17. ISSN 0972-8449.
Szczesna-Skorupa E. & Kemper, B. N-terminal basic amino acids are not required for translocation and processing of preproparathyroid hormone. Mol. Endocrinol. (1989) 174-178.
Tröβe, C., Ravneberg, H., Stern, B. & Pryme. I.F. Vectors encoding seven oikosin signal peptides transfected into CHO cells differ greatly in mediating Gaussia luciferase and human endostatin production although mRNA levels are largely unaffected. Gene Reg. and Systems Biol. 1 (2007) 303-312.
Zhang, L., Leng Q. & Mixson, A.J. Alteration in the IL-2 signal peptide affects secretion of proteins in vitro and in vivo. J. Gene Med. 7 (2005) 354-365.

## Claims

1. A method for the increased production of a recombinant protein of interest in a eukaryotic cell, said method comprising
(i) providing a eukaryotic cell pool containing a final rational library of up to 4¹⁸ different vector variants containing randomised variants of a signal sequence and a gene encoding the recombinant protein of interest, said eukaryotic cell pool being obtained by a method comprising
(a) providing said signal sequence and defining at most 18 nucleotide residues coding for at most 6 amino acid residues at specific positions in said signal sequence to be randomised; and
(b) amplifying said signal sequence as part of a double stranded DNA plasmid, wherein said plasmid may be a preliminary vector, or a final vector containing a gene encoding the recombinant protein of interest, and subjecting said signal sequence to randomisation at said specified positions thereby generating a pool of randomised variants of said signal sequence as part of a vector; and
(ci) amplifying said pool of randomised variants of said signal sequence being part of a preliminary vector, thereby generating a pre-made rational library, and introducing said pool of randomised variants of said signal sequence being part of the pre-made rational library into a recipient vector containing a gene encoding the recombinant protein in a seamless manner, thereby generating the final rational library, or
(cii) amplifying said pool of randomised variants of said signal sequence being part of a preliminary vector, thereby generating a pre-made rational library, and introducing a gene encoding the recombinant protein into the pre-made rational library in a seamless manner, thereby generating the final rational library, or
(ciii) amplifying said pool of randomised variants of said signal sequence being part of a final vector, thereby generating the final rational library; and
(d) transforming said final rational library into eukaryotic cells thereby obtaining said eukaryotic cell pool; and
(ii) culturing said cell pool under conditions suitable for the production of said recombinant protein of interest; and
(iii) identifying within said cell pool a cell clone that produces the recombinant protein of interest at a higher level than that mediated by the unmodified signal sequence.

2. The method for the increased production of a recombinant protein of interest in a eukaryotic cell of claim 1, said method comprising
(i) providing a signal sequence and defining at most 18 nucleotide residues coding for at most 6 amino acid residues at specific positions in said signal sequence to be randomised; and
(ii) amplifying said signal sequence as part of a double stranded DNA plasmid, wherein said plasmid may be a preliminary vector, or a final vector containing a gene encoding the recombinant protein of interest, and subjecting said signal sequence to randomisation at said specified positions thereby generating a pool of randomised variants of said signal sequence as part of a vector; and
(iiia) amplifying said pool of randomised variants of said signal sequence being part of a preliminary vector, thereby generating a pre-made rational library, and introducing said pool of randomised variants of said signal sequence being part of the pre-made rational library into a recipient vector containing a gene encoding the recombinant protein in a seamless manner, thereby generating a final rational library of up to 4¹⁸ different vector variants containing randomised variants of said signal sequence and the gene encoding the recombinant protein of interest, or
(iiib) amplifying said pool of randomised variants of said signal sequence being part of a preliminary vector, thereby generating a pre-made rational library, and introducing a gene encoding the recombinant protein into the pre-made rational library in a seamless manner, thereby generating a final rational library of up to 4¹⁸ different vector variants containing randomised variants of said signal sequence and the gene encoding the recombinant protein of interest, or
(iiic) amplifying said pool of randomised variants of said signal sequence being part of a final vector, thereby generating a final rational library of up to 4¹⁸ different vector variants containing randomised variants of said signal sequence and the gene encoding the recombinant protein of interest; and
(iv) transforming said final rational library into eukaryotic cells thereby obtaining a eukaryotic cell pool containing the final rational library; and
(v) culturing said cell pool under conditions suitable for the production of said recombinant protein of interest; and
(vi) identifying within said cell pool a cell clone that produces the recombinant protein of interest at a higher level than that mediated by the unmodified signal sequence.

3. The method for the increased production of a recombinant protein of interest in a eukaryotic cell of claim 1, wherein said method comprises
(i) providing a pre-made rational library of up to 4¹⁸ different vector variants containing randomised variants of a signal sequence as part of a preliminary vector, said pre-made rational library being obtained by a method comprising:
(a) providing said signal sequence and defining at most 18 nucleotide residues coding for at most 6 amino acid residues at specific positions in said signal sequence to be randomised; and
(b) amplifying said signal sequence as part of a double stranded DNA plasmid being a preliminary vector and subjecting said signal sequence to randomisation at said specified positions thereby generating a pool of randomised variants of said signal sequence as part of a preliminary vector; and
(c) amplifying said pool of randomised variants of said signal sequence as part of a preliminary vector, thereby generating said pre-made rational library; and
(iia) introducing said randomised variants of said signal sequence of said pre-made rational library into a recipient vector containing a gene encoding the recombinant protein in a seamless manner, thereby generating a final rational library of up to 4¹⁸ different vector variants containing randomised variants of said signal sequence and the gene encoding the recombinant protein of interest, or
(iib) introducing a gene encoding the recombinant protein into the pre-made rational library in a seamless manner, thereby generating a final rational library of up to 4¹⁸ different vector variants containing randomised variants of said signal sequence and the gene encoding the recombinant protein of interest; and
(iii) transforming said final rational library into eukaryotic cells thereby obtaining a eukaryotic cell pool containing the final rational library; and
(iv) culturing said cell pool under conditions suitable for the production of said recombinant protein of interest; and
(v) identifying within said cell pool a cell clone that produces the recombinant protein of interest at a higher level than that mediated by the unmodified signal sequence.

4. The method for the increased production of a recombinant protein of interest in a eukaryotic cell of claim 3, wherein said method comprises
(i) providing a signal sequence and defining at most 18 nucleotide residues coding for at most 6 amino acid residues at specific positions in said signal sequence to be randomised; and
(ii) amplifying said signal sequence as part of a double stranded DNA plasmid being a preliminary vector and subjecting said signal sequence to randomisation at said specified positions thereby generating a pool of randomised variants of said signal sequence as part of a preliminary vector; and
(iii) amplifying said pool of randomised variants of said signal sequence as part of a preliminary vector, thereby generating a pre-made rational library of up to 4¹⁸ different vector variants containing randomised variants of said signal sequence as part of a preliminary vector; and
(iva) introducing said randomised variants of said signal sequence of said pre-made rational library into a recipient vector containing a gene encoding the recombinant protein in a seamless manner, thereby generating a final rational library of up to 4¹⁸ different vector variants containing randomised variants of said signal sequence and the gene encoding the recombinant protein of interest, or
(ivb) introducing a gene encoding the recombinant protein into the pre-made rational library in a seamless manner, thereby generating a final rational library of up to 4¹⁸ different vector variants containing randomised variants of said signal sequence and the gene encoding the recombinant protein of interest; and
(v) transforming said final rational library into eukaryotic cells thereby obtaining a eukaryotic cell pool containing the final rational library; and
(vi) culturing said cell pool under conditions suitable for the production of said recombinant protein of interest; and
(vii) identifying within said cell pool a cell clone that produces the recombinant protein of interest at a higher level than that mediated by the unmodified signal sequence.

5. The method of any preceding claim wherein 6, 7, 8, 9, 10, 11 or 12 nucleotides, coding for 2, 3 or 4 amino acid residues, are randomised, preferably wherein 9 or 12 nucleotides coding for 3 or 4 amino acid residues are randomised.

6. The method of any preceding claim, wherein said signal sequence is selected from the group consisting of signal sequences from human, rodent, Gaussia princeps, Metridia longa and Oikopleura dioica.

7. The method of any preceding claim, wherein said signal sequence has the nucleotide sequence as shown in SEQ ID NO: 1, 2, 3, 4, 5, 6, 7, 8, 9, 10, 11 or 12.

8. The method of claim 7, wherein said signal sequence has the nucleotide sequence as shown in SEQ ID NO: 1, 2, 3, 4, 5 or 6.

9. The method of any preceding claim wherein said pre-made rational library is equipped with restriction enzyme recognition sites enabling the seamless insertion of the randomised variant of a signal peptide within said pre-made rational library into any recipient vector encoding a recombinant protein of interest or the insertion of a gene encoding a recombinant protein of interest into the pre-made rational library.

10. The method of any preceding claim, wherein said eukaryotic cells are selected from the group consisting of animal, plant, fungi and yeast cells.

11. The method of claim 10, wherein said animal cell is a mammalian or insect cell.

12. The method of claim 11, wherein said mammalian cell is selected from the group consisting of the cell line families CHO, NSO, SP2/0, 293, myeloma, NOS, COS, BHK, HeLa, Per.C6, and derivatives thereof.

13. The method of any preceding claim, wherein all signal sequences present in any of the vectors have been seamlessly cloned.

14. The method of any preceding claim, wherein said randomised variants of signal sequences are generated either by gene synthesis where random nucleotides are incorporated at specific positions or by Thermal Cycling utilising a mutagenic primer.

15. The method of claim 14, wherein said mutagenic primer used in the Thermal Cycling reaction comprises all randomised nucleotides at all specified positions and has a length between 60 and 100 nucleotides, a total TM from 70 to 85 °C and similar TMs with values from 55 to 70 °C at both non-mutated ends flanking the mutated region.

## Patentansprüche

1. Ein Verfahren für die Produktionssteigerung eines rekombinanten Proteins von Interesse in einer eukaryotischen Zelle, wobei besagtes Verfahren umfasst
(i) Bereitstellung eines eukaryotischen Zellpools, welches eine endgültige rationale Bibliothek von bis zu 4¹⁸ verschiedenen Vektorvarianten enthält, welche randomisierte Varianten einer Signalsequenz enthalten und ein Gen, das für das rekombinante Protein von Interesse kodiert, wobei besagter eukaryotischer Zellpool durch ein Verfahren erhalten wird, das umfasst
(a) Bereitstellung der besagten Signalsequenz und Festlegung von höchstens 18 Nukleotidresten, die für höchstens 6 Aminosäurereste kodieren, an bestimmten Positionen in besagter Signalsequenz, die zu randomiseren sind; und
(b) Vervielfältigung der besagten Signalsequenz als Teil eines DNA-Doppelstrang-Plasmids, wobei besagtes Plasmid ein vorläufiger Vektor sein kann oder ein endgültiger Vektor, welcher ein Gen enthält, das für das rekombinante Protein von Interesse kodiert und Randomisierung der besagten Signalsequenz an besagten festgelegten Positionen, wodurch ein Pool an randomisierten Varianten der besagten Signalsequenz als Teil eines Vektors erzeugt wird; und
(ci) Vervielfältigung des besagten Pools an randomisierten Varianten der besagten Signalsequenz, welche Teil eines vorläufigen Vektors ist, wodurch eine vorgefertigte rationale Bibliothek erzeugt wird und Einbringen des besagten Pools an randomisierten Varianten der besagten Signalsequenz, welche Teil einer vorgefertigten rationalen Bibliothek ist, in einen Empfängervektor, welcher ein Gen enthält, das für das rekombinante Protein von Interesse kodiert, in einer nahtlosen Art und Weise, wodurch die endgültige rationale Bibliothek erzeugt wird, oder
(cii) Vervielfältigung des besagten Pools an randomisierten Varianten der besagten Signalsequenz, welche Teil eines vorläufigen Vektors ist, wodurch und eine vorgefertigte rationale Bibliothek erzeugt wird und Einbringen eines Gens, das für das rekombinante Protein kodiert, in die vorgefertigte rationale Bibliothek in einer nahtlosen Art und Weise, wodurch die endgültige rationale Bibliothek erzeugt wird, oder
(ciii) Vervielfältigung des besagten Pools an randomisierten Varianten der besagten Signalsequenz, welche Teil eines endgültigen Vektors ist, wodurch und die endgültige rationale Bibliothek erzeugt wird; und
(d) Transformieren der besagten endgültigen rationalen Bibliothek in eine eukaryotische Zelle, wodurch der besagte eukaryotische Zellpool erhalten wird; und
(ii) Kultivierung des besagten Zellpools unter Bedingungen, die für die Produktion des besagten rekombinanten Proteins von Interesse geeignet sind; und
(iii) Identifizierung in besagtem Zellpool eines Zellklons, welcher das rekombinante Protein von Interesse in einer größeren Menge produziert als mittels der nicht modifizierten Signalsequenz.

2. Das Verfahren für die Produktionssteigerung eines rekombinanten Proteins von Interesse in einer eukaryotischen Zelle nach Anspruch 1, wobei besagtes Verfahren umfasst
(i) Bereitstellung einer Signalsequenz und Festlegung von höchstens 18 Nukleotidresten, die für höchstens 6 Aminosäurereste kodieren, an bestimmten Positionen in besagter Signalsequenz, die zu randomiseren sind; und
(ii) Vervielfältigung der besagten Signalsequenz als Teil eines DNA-Doppelstrang-Plasmids, wobei besagtes Plasmid ein vorläufiger Vektor sein kann oder ein endgültiger Vektor, welcher ein Gen enthält, das für das rekombinante Protein von Interesse kodiert und Randomisierung der besagten Signalsequenz an besagten festgelegten Positionen, wodurch ein Pool an randomisierten Varianten der besagten Signalsequenz als Teil eines Vektors erzeugt wird; und
(iiia) Vervielfältigung des besagten Pools an randomisierten Varianten der besagten Signalsequenz, welche Teil eines vorläufigen Vektors ist, wodurch eine vorgefertigte rationalen Bibliothek erzeugt wird und Einbringen des besagten Pools an randomisierten Varianten der besagten Signalsequenz, welche Teil einer vorgefertigten rationalen Bibliothek ist, in einen Empfängervektor, welcher ein Gen enthält, das für das rekombinante Protein von Interesse kodiert, in einer nahtlosen Art und Weise, wodurch eine endgültige rationale Bibliothek von bis zu 4¹⁸ verschiedenen Vektorvarianten erzeugt wird, welche randomisierte Varianten der besagten Signalsequenz enthalten und das Gen, das für das rekombinante Protein von Interesse kodiert, oder
(iiib) Vervielfältigung des besagten Pools an randomisierten Varianten der besagten Signalsequenz, welche Teil eines vorläufigen Vektors ist, wodurch eine vorgefertigte rationale Bibliothek erzeugt wird und Einbringen eines Gens, das für das rekombinante Protein kodiert, in die vorgefertigte rationale Bibliothek in einer nahtlosen Art und Weise, wodurch und eine endgültige rationale Bibliothek von bis zu 4¹⁸ verschiedenen Vektorvarianten erzeugt wird, welche randomisierte Varianten der besagten Signalsequenz enthalten und das Gen, das für das rekombinante Protein von Interesse kodiert, oder
(iiic) Vervielfältigung des besagten Pools an randomisierten Varianten der besagten Signalsequenz, welche Teil eines endgültigen Vektors ist, wodurch eine endgültige rationale Bibliothek von bis zu 4¹⁸ verschiedenen Vektorvarianten erzeugt wird, welche randomisierte Varianten der besagten Signalsequenz enthalten und das Gen, das für das rekombinante Protein von Interesse kodiert; und
(iv) Transformieren der besagten endgültigen rationalen Bibliothek in eine eukaryotische Zelle, wodurch ein eukaryotischer Zellpool erhalten wird, welches die endgültige rationale Bibliothek enthält; und
(v) Kultivierung des besagten Zellpools unter Bedingungen, die für die Produktion des besagten rekombinanten Proteins von Interesse geeignet sind; und
(vi) Identifizierung in besagtem Zellpool eines Zellklons, welcher das rekombinante Protein von Interesse in einer größeren Menge produziert als mittels Verwendung der nicht modifizierten Signalsequenz.

3. Das Verfahren für die Produktionssteigerung eines rekombinanten Proteins von Interesse in einer eukaryotischen Zelle nach Anspruch 1, wobei besagtes Verfahren umfasst
(i) Bereitstellung einer vorgefertigten rationalen Bibliothek von bis zu 4¹⁸ verschiedenen Vektorvarianten, welche randomisierte Varianten einer Signalsequenz als Teil eines vorläufigen Vektors enthalten, wobei besagte vorgefertigte rationale Bibliothek durch ein Verfahren erhalten wird, das umfasst
(a) Bereitstellung der besagten Signalsequenz und Festlegung von höchstens 18 Nukleotidresten, die für höchstens 6 Aminosäurereste kodieren, an bestimmten Positionen in besagter Signalsequenz, die zu randomiseren sind; und
(b) Vervielfältigung der besagten Signalsequenz als Teil eines DNA-Doppelstrang-Plasmids, welches ein vorläufiger Vektor ist und Randomisierung der besagten Signalsequenz an besagten festgelegten Positionen, wodurch ein Pool an randomisierten Varianten der besagten Signalsequenz als Teil eines vorläufigen Vektors erzeugt wird; und
(c) Vervielfältigung des besagten Pools an randomisierten Varianten der besagten Signalsequenz als Teil eines vorläufigen Vektors, wodurch besagte vorgefertigte rationale Bibliothek erzeugt wird; und
(iia) Einbringen besagter randomisierter Varianten der besagten Signalsequenz der besagten vorgefertigten rationalen Bibliothek in einen Empfängervektor, welcher ein Gen enthält, das für das rekombinante Protein von Interesse kodiert, in einer nahtlosen Art und Weise, wodurch eine endgültige rationale Bibliothek von bis zu 4¹⁸ verschiedenen Vektorvarianten erzeugt wird, welche randomisierte Varianten der besagten Signalsequenz enthalten und das Gen, das für das rekombinante Protein von Interesse kodiert, oder
(iib) Einbringen eines Gens, das für das rekombinante Protein kodiert, in die vorgefertigte rationale Bibliothek in einer nahtlosen Art und Weise, wodurch eine endgültige rationale Bibliothek von bis zu 4¹⁸ verschiedenen Vektorvarianten erzeugt wird, welche randomisierte Varianten der besagten Signalsequenz enthalten und das Gen, das für das rekombinante Protein von Interesse kodiert; und
(iii) Transformieren der besagten endgültigen rationalen Bibliothek in eine eukaryotische Zelle, wodurch ein eukaryotischer Zellpool erhalten wird, welches die endgültige rationale Bibliothek enthält; und
(iv) Kultivierung des besagten Zellpools unter Bedingungen, die für die Produktion des besagten rekombinanten Proteins von Interesse geeignet sind; und
(v) Identifizierung in besagtem Zellpool eines Zellklons, welcher das rekombinante Protein von Interesse in einer größeren Menge produziert als mittels Verwendung der nicht modifizierten Signalsequenz.

4. Das Verfahren für die Produktionssteigerung eines rekombinanten Proteins von Interesse in einer eukaryotischen Zelle nach Anspruch 3, wobei besagtes Verfahren umfasst
(i) Bereitstellung einer Signalsequenz und Festlegung von höchstens 18 Nukleotidresten, die für höchstens 6 Aminosäurereste kodieren, an bestimmten Positionen in besagter Signalsequenz, die zu randomiseren sind; und
(ii) Vervielfältigung der besagten Signalsequenz als Teil eines DNA-Doppelstrang-Plasmids, welches ein vorläufiger Vektor ist und Randomisierung der besagten Signalsequenz an besagten festgelegten Positionen, wodurch ein Pool an randomisierten Varianten der besagten Signalsequenz als Teil eines vorläufigen Vektors erzeugt wird; und
(iii) Vervielfältigung des besagten Pools an randomisierten Varianten der besagten Signalsequenz als Teil eines vorläufigen Vektors, wodurch eine vorgefertigte rationale Bibliothek von bis zu 4¹⁸ verschiedenen Vektorvarianten erzeugt wird, welche randomisierte Varianten der besagten Signalsequenz als Teil eines vorläufigen Vektors enthalten; und
(iva) inbringen besagter randomisierter Varianten der besagten Signalsequenz der besagten vorgefertigten rationalen Bibliothek in einen Empfängervektor, welcher ein Gen enthält, das für das rekombinante Protein von Interesse kodiert, in einer nahtlosen Art und Weise, wobei eine endgültige rationale Bibliothek von bis zu 4¹⁸ verschiedenen Vektorvarianten erzeugt wird, welche randomisierte Varianten der besagten Signalsequenz enthalten und das Gen, das für das rekombinante Protein von Interesse kodiert, oder
(ivb) Einbringen eines Gens, das für das rekombinante Protein kodiert, in die vorgefertigte rationale Bibliothek in einer nahtlosen Art und Weise, wobei eine endgültige rationale Bibliothek von bis zu 4¹⁸ verschiedenen Vektorvarianten erzeugt wird, welche randomisierte Varianten der besagten Signalsequenz enthalten und das Gen, das für das rekombinante Protein von Interesse kodiert; und
(v) Transformieren der besagten endgültigen rationalen Bibliothek in eine eukaryotische Zelle, wodurch ein eukaryotischer Zellpool erhalten wird, welches die endgültige rationale Bibliothek enthält; und
(vi) Kultivierung des besagten Zellpools unter Bedingungen, die für die Produktion des besagten rekombinanten Proteins von Interesse geeignet sind; und
(vii) Identifizierung in besagtem Zellpool eines Zellklons, welcher das rekombinante Protein von Interesse in einer größeren Menge produziert als mittels Verwendung der nicht modifizierten Signalsequenz.

5. Das Verfahren gemäß einem der vorhergehenden Ansprüche, wobei 6, 7, 8, 9, 10, 11 oder 12 Nukleotide, die für 2, 3 oder 4 Aminosäurereste kodieren, randomisiert sind, wobei vorzugsweise 9 oder 12 Nukleotide, die für 3 oder 4 Aminosäurereste kodieren, randomisiert sind.

6. Das Verfahren gemäß einem der vorhergehenden Ansprüche, wobei besagte Signalsequenz aus der Gruppe ausgewählt ist, die aus Signalsequenzen besteht, die vom Menschen, von agetieren, Gaussia princeps, Metridia longa und Oikopleura dioica herstammen.

7. Das Verfahren gemäß einem der vorhergehenden Ansprüche, wobei besagte Signalsequenz die Nukleotidsequenz hat wie in SEQ ID NO: 1, 2, 3, 4, 5, 6, 7, 8, 9, 10, 11 oder 12 gezeigt.

8. Das Verfahren gemäß Anspruch 7, wobei besagte Signalsequenz die Nukleotidsequenz hat wie in SEQ ID NO: 1, 2, 3, 4, 5 oder 6 gezeigt.

9. Das Verfahren gemäß einem der vorhergehenden Ansprüche, wobei besagte vorgefertigte rationale Bibliothek mit Restriktionsenzym-Erkennungsstellen ausgestattet ist, welche die nahtlose Insertion der randomisierten Varianten einer Signalsequenz in besagter vorgefertigter rationaler Bibliothek in jeden Empfängervektor, welcher für ein rekombinantes Protein von Interesse kodiert, ermöglichen oder die Insertion eines Gens, das für ein rekombinante Protein von Interesse kodiert, in die vorgefertigte rationale Bibliothek.

10. Das Verfahren gemäß einem der vorhergehenden Ansprüche, wobei besagte eukaryotischen Zellen aus der Gruppe ausgewählt sind, die aus Tier-, Pflanzen-, Pilz- und Hefezellen besteht.

11. Das Verfahren gemäß Anspruch 10, wobei besagte Tierzelle eine Säugetier- oder Insektenzelle ist.

12. Das Verfahren gemäß Anspruch 11, wobei besagte Säugetierzelle aus der Gruppe ausgewählt ist, die aus den Zelllinie-Familien CHO, NSO, SP2/0, 293, Myelomzellinie, NOS, COS, BHK, HeLa, Per.C6 und Derivaten davon besteht.

13. Das Verfahren gemäß einem der vorhergehenden Ansprüche, wobei alle Signalsequenzen, welche in einem der Vektoren enthalten sind, nahtlos in diese kloniert wurden.

14. Das Verfahren gemäß einem der vorhergehenden Ansprüche, wobei besagte randomisierte Varianten der Signalsequenzen entweder durch Genesynthese erzeugt werden, bei der zufällige Nukleotide an bestimmten Positionen eingefügt werden oder durch "Thermal Cycling" unter Verwendung eines mutagenen Primers.

15. Das Verfahren gemäß Anspruch 14, wobei besagter mutagene Primer, der in der Thermal Cycling Reaktion verwendet wird, alle randomisierten Nukleotide in allen festgelegten Positionen enthält und eine Länge von 60 bis 100 Nukleotiden, eine Gesamt-Schmelztemperatur (TM) von 70 bis 85 °C und ähnliche TMs mit Werten von 55 bis 70 °C an beiden nicht-mutierten Enden, welche die mutierte Region flankieren, aufweist.

## Revendications

1. Procédé pour la production accrue d'une protéine recombinante d'intérêt dans une cellule eucaryote, ledit procédé comprenant
(i) la fourniture d'un groupe de cellules eucaryotes contenant une banque rationnelle finale de jusqu'à 4¹⁸ variants de vecteurs différents contenant des variants randomisés d'une séquence signal et un gène codant pour la protéine recombinante d'intérêt, ledit groupe de cellules eucaryotes étant obtenu par un procédé comprenant
(a) la fourniture de ladite séquence signal et la définition au plus de 18 résidus nucléotidiques codant pour au plus 6 résidus d'acides aminés au niveau de positions spécifiques dans ladite séquence signal à randomiser ; et
(b) l'amplification de ladite séquence signal comme partie d'un plasmide d'ADN double brin, dans lequel ledit plasmide peut être un vecteur préliminaire, ou un vecteur final contenant un gène codant pour la protéine recombinante d'intérêt, et la soumission de ladite séquence signal à une randomisation aux niveaux desdites positions spécifiées générant de cette façon un groupe de variants randomisés de ladite séquence signal comme partie d'un vecteur ; et
(ci) l'amplification dudit groupe de variants randomisés de ladite séquence signal faisant partie d'un vecteur préliminaire, générant de cette façon une banque rationnelle préfabriquée, et l'introduction dudit groupe de variants randomisés de ladite séquence signal faisant partie de la banque rationnelle préfabriquée dans un vecteur receveur contenant un gène codant pour la protéine recombinante de façon continue, générant de cette façon la banque rationnelle finale, ou
(cii) l'amplification dudit groupe de variants randomisés de ladite séquence signal faisant partie d'un vecteur préliminaire, générant de cette façon une banque rationnelle préfabriquée, et l'introduction d'un gène codant pour la protéine recombinante dans la banque rationnelle préfabriquée de façon continue, générant de cette façon la banque rationnelle finale, ou
(ciii) l'amplification dudit groupe de variants randomisés de ladite séquence signal faisant partie d'un vecteur final, générant de cette façon la banque rationnelle finale ; et
(d) la transformation de ladite banque rationnelle finale dans des cellules eucaryotes obtenant de cette façon ledit groupe de cellules eucaryotes ; et
(ii) la culture dudit groupe de cellules dans des conditions appropriées pour la production de ladite protéine recombinante d'intérêt ; et
(iii) l'identification au sein dudit groupe de cellules d'un clone de cellules qui produit la protéine recombinante d'intérêt à un taux supérieur à celui médié par la séquence signal non modifiée.

2. Procédé pour la production accrue d'une protéine recombinante d'intérêt dans une cellule eucaryote selon la revendication 1, ledit procédé comprenant
(i) la fourniture d'une séquence signal et la définition au plus de 18 résidus nucléotidiques codant pour au plus 6 résidus d'acides aminés au niveau de positions spécifiques dans ladite séquence signal à randomiser ; et
(ii) l'amplification de ladite séquence signal comme partie d'un plasmide d'ADN double brin, dans lequel ledit plasmide peut être un vecteur préliminaire, ou un vecteur final contenant un gène codant pour la protéine recombinante d'intérêt, et la soumission de ladite séquence signal à une randomisation aux niveaux desdites positions spécifiées générant de cette façon un groupe de variants randomisés de ladite séquence signal comme partie d'un vecteur ; et
(iiia) l'amplification dudit groupe de variants randomisés de ladite séquence signal faisant partie d'un vecteur préliminaire, générant de cette façon une banque rationnelle préfabriquée, et l'introduction dudit groupe de variants randomisés de ladite séquence signal faisant partie de la banque rationnelle préfabriquée dans un vecteur receveur contenant un gène codant pour la protéine recombinante de façon continue, générant de cette façon une banque rationnelle finale de jusqu'à 4¹⁸ variants de vecteurs différents contenant des variants randomisés de ladite séquence signal et le gène codant pour la protéine recombinante d'intérêt, ou
(iiib) l'amplification dudit groupe de variants randomisés de ladite séquence signal faisant partie d'un vecteur préliminaire, générant de cette façon une banque rationnelle préfabriquée, et l'introduction d'un gène codant pour la protéine recombinante dans la banque rationnelle préfabriquée de façon continue, générant de cette façon une banque rationnelle finale de jusqu'à 4¹⁸ variants de vecteurs différents contenant des variants randomisés de ladite séquence signal et le gène codant pour la protéine recombinante d'intérêt, ou
(iiic) l'amplification dudit groupe de variants randomisés de ladite séquence signal faisant partie d'un vecteur final, générant de cette façon une banque rationnelle finale de jusqu'à 4¹⁸ variants de vecteurs différents contenant des variants randomisés de ladite séquence signal et le gène codant pour la protéine recombinante d'intérêt ; et
(iv) la transformation de ladite banque rationnelle finale dans des cellules eucaryotes obtenant de cette façon un groupe de cellules eucaryotes contenant la banque rationnelle finale ; et
(v) la culture dudit groupe de cellules dans des conditions appropriées pour la production de ladite protéine recombinante d'intérêt ; et
(vi) l'identification au sein dudit groupe de cellules d'un clone de cellules qui produit la protéine recombinante d'intérêt à un taux supérieur à celui médié par la séquence signal non modifiée.

3. Procédé pour la production accrue d'une protéine recombinante d'intérêt dans une cellule eucaryote selon la revendication 1, dans lequel ledit procédé comprend
(i) la fourniture d'une banque rationnelle préfabriquée de jusqu'à 4¹⁸ variants de vecteurs différents contenant des variants randomisés d'une séquence signal comme partie d'un vecteur préliminaire, ladite banque rationnelle préfabriquée étant obtenue par un procédé comprenant :
(a) la fourniture de ladite séquence signal et la définition au plus de 18 résidus nucléotidiques codant pour au plus 6 résidus d'acides aminés au niveau de positions spécifiques dans ladite séquence signal à randomiser ; et
(b) l'amplification de ladite séquence signal comme partie d'un plasmide d'ADN double brin étant un vecteur préliminaire et la soumission de ladite séquence signal à une randomisation aux niveaux desdites positions spécifiées générant de cette façon un groupe de variants randomisés de ladite séquence signal comme partie d'un vecteur préliminaire ; et
(c) l'amplification dudit groupe de variants randomisés de ladite séquence signal comme partie d'un vecteur préliminaire, générant de cette façon ladite banque rationnelle préfabriquée ; et
(iia) l'introduction desdits variants randomisés de ladite séquence signal de ladite banque rationnelle préfabriquée dans un vecteur receveur contenant un gène codant pour la protéine recombinante de façon continue, générant de cette façon une banque rationnelle finale de jusqu'à 4¹⁸ variants de vecteurs différents contenant des variants randomisés de ladite séquence signal et le gène codant pour la protéine recombinante d'intérêt, ou
(iib) l'introduction d'un gène codant pour la protéine recombinante dans la banque rationnelle préfabriquée de façon continue, générant de cette façon une banque rationnelle finale de jusqu'à 4¹⁸ variants de vecteurs différents contenant des variants randomisés de ladite séquence signal et le gène codant pour la protéine recombinante d'intérêt ; et
(iii) la transformation de ladite banque rationnelle finale dans des cellules eucaryotes obtenant de cette façon un groupe de cellules eucaryotes contenant la banque rationnelle finale ; et
(iv) la culture dudit groupe de cellules dans des conditions appropriées pour la production de ladite protéine recombinante d'intérêt ; et
(v) l'identification au sein dudit groupe de cellules d'un clone de cellules qui produit la protéine recombinante d'intérêt à un taux supérieur à celui médié par la séquence signal non modifiée.

4. Procédé pour la production accrue d'une protéine recombinante d'intérêt dans une cellule eucaryote selon la revendication 3, dans lequel ledit procédé comprend
(i) la fourniture d'une séquence signal et la définition au plus de 18 résidus nucléotidiques codant pour au plus 6 résidus d'acides aminés au niveau de positions spécifiques dans ladite séquence signal à randomiser ; et
(ii) l'amplification de ladite séquence signal comme partie d'un plasmide d'ADN double brin étant un vecteur préliminaire et la soumission de ladite séquence signal à une randomisation aux niveaux desdites positions spécifiées générant de cette façon un groupe de variants randomisés de ladite séquence signal comme partie d'un vecteur préliminaire ; et
(iii) l'amplification dudit groupe de variants randomisés de ladite séquence signal comme partie d'un vecteur préliminaire, générant de cette façon une banque rationnelle préfabriquée de jusqu'à 4¹⁸ variants de vecteurs différents contenant des variants randomisés de ladite séquence signal comme partie d'un vecteur préliminaire ; et
(iva) l'introduction desdits variants randomisés de ladite séquence signal de ladite banque rationnelle préfabriquée dans un vecteur receveur contenant un gène codant pour la protéine recombinante de façon continue, générant de cette façon une banque rationnelle finale de jusqu'à 4¹⁸ variants de vecteurs différents contenant des variants randomisés de ladite séquence signal et le gène codant pour la protéine recombinante d'intérêt, ou
(ivb) l'introduction d'un gène codant pour la protéine recombinante dans la banque rationnelle préfabriquée de façon continue, générant de cette façon une banque rationnelle finale de jusqu'à 4¹⁸ variants de vecteurs différents contenant des variants randomisés de ladite séquence signal et le gène codant pour la protéine recombinante d'intérêt ; et
(v) la transformation de ladite banque rationnelle finale dans des cellules eucaryotes obtenant de cette façon un groupe de cellules eucaryotes contenant la banque rationnelle finale ; et
(vi) la culture dudit groupe de cellules dans des conditions appropriées pour la production de ladite protéine recombinante d'intérêt ; et
(vii) l'identification au sein dudit groupe de cellules d'un clone de cellules qui produit la protéine recombinante d'intérêt à un taux supérieur à celui médié par la séquence signal non modifiée.

5. Procédé selon une quelconque revendication précédente dans lequel 6, 7, 8, 9, 10, 11 ou 12 nucléotides, codant pour 2, 3 ou 4 résidus d'acides aminés, sont randomisés, de préférence dans lequel 9 ou 12 nucléotides codant pour 3 ou 4 résidus d'acides aminés sont randomisés.

6. Procédé selon une quelconque revendication précédente, dans lequel ladite séquence signal est choisie dans le groupe constitué de séquences signal de l'être humain, de rongeur, de Gaussia princeps, de Metridia longa et d'Oikopleura dioica.

7. Procédé selon une quelconque revendication précédente, dans lequel ladite séquence signal a la séquence nucléotidique telle que représentée par SEQ ID NO : 1, 2, 3, 4, 5, 6, 7, 8, 9, 10, 11 ou 12.

8. Procédé selon la revendication 7, dans lequel ladite séquence signal a la séquence nucléotidique telle que représentée par SEQ ID NO : 1, 2, 3, 4, 5 ou 6.

9. Procédé selon une quelconque revendication précédente dans lequel ladite banque rationnelle préfabriquée est équipée de sites de reconnaissance d'enzymes de restriction permettant l'insertion continue du variant randomisé d'un peptide signal au sein de ladite banque rationnelle préfabriquée dans un vecteur receveur quelconque codant pour une protéine recombinante d'intérêt ou l'insertion d'un gène codant pour une protéine recombinante d'intérêt dans la banque rationnelle préfabriquée.

10. Procédé selon une quelconque revendication précédente, dans lequel lesdites cellules eucaryotes sont choisies dans le groupe constitué de cellules animales, végétales, fongiques et de levures.

11. Procédé selon la revendication 10, dans lequel ladite cellule animale est une cellule de mammifère ou d'insecte.

12. Procédé selon la revendication 11, dans lequel ladite cellule de mammifère est choisie dans le groupe constitué des familles de lignées cellulaires CHO, NSO, SP2/0, 293, de myélome, NOS, COS, BHK, HeLa, Per.C6, et leurs dérivés.

13. Procédé selon une quelconque revendication précédente, dans lequel toutes les séquences signal présentes dans l'un quelconque des vecteurs ont été clonées de façon continue.

14. Procédé selon une quelconque revendication précédente, dans lequel lesdits variants randomisés des séquences signal sont générés soit par synthèse de gènes où des nucléotides aléatoires sont incorporés au niveau de positions spécifiques soit par thermocyclage en utilisant une amorce mutagène.

15. Procédé selon la revendication 14, dans lequel ladite amorce mutagène utilisée dans la réaction de thermocyclage comprend tous les nucléotides randomisés au niveau de toutes les positions spécifiées et a une longueur située entre 60 et 100 nucléotides, une TM totale de 70 à 85 °C et des TM similaires avec des valeurs de 55 à 70 °C aux deux extrémités non mutées flanquant la région mutée.
